# EUROPÄISCHE PATENTSCHRIFT

(11) **EP 1 097 998 B1**
(45) Veröffentlichungstag und Bekanntmachung des Hinweises auf die Patenterteilung: **07.05.2008**
(21) Anmeldenummer: 00122056.5
(22) Anmeldetag: 11.10.2000
(51) Int. Cl.: C12N 15/77, C12P 13/04, C12P 13/08, C12P 13/02

(54) **Plasmide aus Corynebacterium glutamicum und deren Verwendung**
Plasmids of Corynebacterium glutamicum and the use thereof
Plasmides de Corynebacterium glutamicum et leur utilisation

(30) Priorität: 05.11.1999 DE 19953206
(43) Veröffentlichungstag der Anmeldung: 09.05.2001
(73) Patentinhaber: Evonik Degussa GmbH, 40474 Düsseldorf (DE)
(72) Erfinder: Tauch, Andreas, 33647 Bielefeld (DE); Kalinowski, Jörn, Dr., 33615 Bielefeld (DE); Pühler, Alfred, Prof. Dr., 33739 Bielefeld (DE); Thierbach, Georg, Dr., 33613 Bielefeld (DE)

(56) Entgegenhaltungen:
- EP-A- 0 472 869
- WO-A-95/19442
- DATABASE EMBL [Online] accession: Y14748, 8. September 1997 (1997-09-08) NESVERA J ET AL: "Corynebacterium glutamicum plasmid pCG4 integron (InCg) sequence" XP002159885

### SEQUENZPROTOKOLL

<110> Degussa-Hüls AG
<120> Neue Plasmide aus Corynebacterium glutamicum und deren Verwendung
<130> 990111 BT
<140>
   <141>
<160> 10
<170> PatentIn Ver. 2.1
<210> 1
   <211> 4539
   <212> DNA
   <213> Corynebacterium glutamicum LP-6
<220>
   <221> CDS
   <222> (228)..(824)
   <223> parA
<220>
   <221> CDS
   <222> (1829)..(3295)
   <223> repA
<400> 1
   cctaggctta gatgtgctgt cataattttc gcccctcccg tcagacattt ggacatggat 60
   ctcgggaaag attaagcggg ggaacttgaa ataattccac tttaaactag gaaatagcag 120
   gtcaaagcga tatgttaagg cgctataaca tgagtatgtt atagcgctaa aacacacaac 180
   atacttatgt tatgcagcta agacggaagt atgtcagact gataatc atg tcc ata   236
                                                       Met Ser Ile
                                                        1
   ctg act atc gct cac aca aaa ggc ggc gtg gga aaa acg acc tct gca   284
   Leu Thr Ile Ala His Thr Lys Gly Gly Val Gly Lys Thr Thr Ser Ala
        5                   10                  15
   gtg ctt ttg tgt gca gct gcc cac gcc cgc gga cta gcc gtt acc ctc   332
   Val Leu Leu Cys Ala Ala Ala His Ala Arg Gly Leu Ala Val Thr Leu
    20                  25                  30                  35
   att gac tcc gac gct cag ggc acc gcc acc gcc tgg gcc cac gct gcc   380
   Ile Asp Ser Asp Ala Gln Gly Thr Ala Thr Ala Trp Ala His Ala Ala
                    40                  45                  50
   gaa gaa gcc ggt gat act ttc ccg tgg cct atc atc aca gcg gcc acg   428
   Glu Glu Ala Gly Asp Thr Phe Pro Trp Pro Ile Ile Thr Ala Ala Thr
                55                  60                  65
   cct gcc cac ctt gcc cga acg ctc gac ggc cat aac gga ctc gtc atc   476
   Pro Ala His Leu Ala Arg Thr Leu Asp Gly His Asn Gly Leu Val Ile
           70                  75                  80
   gtt gat acc ccg ccc ggt ggc tac gaa gtg atc gag acc gcc atc gaa   524
   Val Asp Thr Pro Pro Gly Gly Tyr Glu Val Ile Glu Thr Ala Ile Glu
       85                  90                  95
   gca ggc gac ctc atc ctg atc ccc acc tct gcc tcc ccg cta gac atc   572
   Ala Gly Asp Leu Ile Leu Ile Pro Thr Ser Ala Ser Pro Leu Asp Ile
   100                 105                 110                 115
   aat cga gtc tgg cca act gtt gag gcc acc agt cac aag ccc gcc gta   620
   Asn Arg Val Trp Pro Thr Val Glu Ala Thr Ser His Lys Pro Ala Val
                   120                 125                 130
   gtc tgt cta tcc cag atc gac tcc cgc acc acc ttg ccc aag att gcc   668
   Val Cys Leu Ser Gln Ile Asp Ser Arg Thr Thr Leu Pro Lys Ile Ala
               135                 140                 145
   cgt acc gca ctt gaa aac gaa ggc gtc gtc gtc gct gaa acg gag atc   716
   Arg Thr Ala Leu Glu Asn Glu Gly Val Val Val Ala Glu Thr Glu Ile
           150                 155                 160
   ccc gcc cga gaa gcg tta aga cat atg tat gct aca act cca caa cgt   764
   Pro Ala Arg Glu Ala Leu Arg His Met Tyr Ala Thr Thr Pro Gln Arg
       165                 170                 175
   tta tat ggc tac gat gaa cta ctt act gaa cta ctg tcc tca aac ctg   812
   Leu Tyr Gly Tyr Asp Glu Leu Leu Thr Glu Leu Leu Ser Ser Asn Leu
   180                 185                 190                 195
   ttg gga gaa cat taatggctga tttatccaag aagctggcga aagcgcccgt      864
   Leu Gly Glu His
   taccagcgcg ccgcaaaaga aagtggccga gacctttact accgccacgg agaagcccac 924
   ccgcaccacc atttaccttc caacaagtct cgctaagcgc cttaagcatg ctgccgtcga 984
   agaggagcgc agtgtctctg caatcctcgc aggactggcc gaagactggc taaacaaaga 1044
   agacgactaa gtatgtttat atgtcatggc atacgacata caaacataac aacatagaaa 1104
   cctaacaacg tattaactcc aagtagtcag cgctggagac catgccccat cgacacgcgg 1164
   ctgcgctgct cgtggggcca gaagtggcgc aggactgatg aaagaaaccc acaagacgtt 1224
   taagcgtcaa agcgtcaagc ggacacaaca tgaaaacatc aagacgttta gacccttgcg 1284
   gcctttgacg ccagtcccgc cgaaacttat acattcgacg caatctatga agagatttaa 1344
   ggaatgaagg agacggcttt aatgtcacca gcacggcgca gttcaagaac cactaccgga 1404
   cgaaaaacaa cacgcccctc ggcggcaaca cccacaccga ccgacgagga aggcacagag 1464
   cttacccggc gtacaaccat ttacctcaaa gaggaaacat ggaaatccat gaagcgcatg 1524
   accgtagaaa caggagaaag cgtttctgcg tacatcgaac gactcattga taaagatgta 1584
   aagcgcgtcc agaaaaagtt actccaaaac cccaaatcgc tataacacga aaacataaca 1644
   acgttatagc gctttagcac taaactgtat cggggcaggt taaaaacttt tcgtgtcgca 1704
   ggcacagagc aatcacactc gtgttactct ggtcgaaaac cttataaatg catgaagtcc 1764
   gccaggcttg cacccctgac ggacttcgct atcacccgga ggacacccgg gggaaagcac 1824
   gtca atg agc tta cct tca aaa gga cga tca agc aca cct aca ggt gtg  1873
        Met Ser Leu Pro Ser Lys Gly Arg Ser Ser Thr Pro Thr Gly Val
        200                 205                 210
   cgt gtt gcc caa cca ctg ccc acc cac cgc gac act ggc ggc ctg gac   1921
   Arg Val Ala Gln Pro Leu Pro Thr His Arg Asp Thr Gly Gly Leu Asp
   215                 220                 225                 230
   gac acc ccg gca gga ttc act gat cgt gat gca ctt ata gat cat ctc   1969
   Asp Thr Pro Ala Gly Phe Thr Asp Arg Asp Ala Leu Ile Asp His Leu
                   235                 240                 245
   ggg cgt aaa gca atc cac gga agc aaa gac cgt gac ttc ggc aaa gct   2017
   Gly Arg Lys Ala Ile His Gly Ser Lys Asp Arg Asp Phe Gly Lys Ala
               250                 255                 260
   tat tac cgc cac gag gac ggt act ctt cgc ccg cgc ttg tat cgc gtg   2065
   Tyr Tyr Arg His Glu Asp Gly Thr Leu Arg Pro Arg Leu Tyr Arg Val
           265                 270                 275
   gat tct gag gcg ttg aca cgc tgc cag tac gtc atg ctc acc acg cag   2113
   Asp Ser Glu Ala Leu Thr Arg Cys Gln Tyr Val Met Leu Thr Thr Gln
       280                 285                 290
   caa tac gcc gct gta tta gtg gtc gat att gac cag ccc ggc caa tcg   2161
   Gln Tyr Ala Ala Val Leu Val Val Asp Ile Asp Gln Pro Gly Gln Ser
   295                 300                 305                 310
   gga ggg cac cca gcg aac tta tcg cct gag gtt cgt cag aag atg gcc   2209
   Gly Gly His Pro Ala Asn Leu Ser Pro Glu Val Arg Gln Lys Met Ala
                   315                 320                 325
   gct ctt atc gag cac aac ctt ggg ccg tcg tgg gtg ggc att aat ccc   2257
   Ala Leu Ile Glu His Asn Leu Gly Pro Ser Trp Val Gly Ile Asn Pro
               330                 335                 340
   caa aac ggt aaa gca cag gcg atc tgg ttg att gat ccg gtg tac gca   2305
   Gln Asn Gly Lys Ala Gln Ala Ile Trp Leu Ile Asp Pro Val Tyr Ala
           345                 350                 355
   gac aaa agc ggc aaa tct cgg cat atg agt ctg ctt gcc gcg acg agc   2353
   Asp Lys Ser Gly Lys Ser Arg His Met Ser Leu Leu Ala Ala Thr Ser
       360                 365                 370
   cgt gct ttg ggt gag ctg ttg gat cat gat ccg aat ttc tct cac cgt   2401
   Arg Ala Leu Gly Glu Leu Leu Asp His Asp Pro Asn Phe Ser His Arg
   375                 380                 385                 390
   ttt agt cgg agc ccg ttt tat gac ggc aac gac cct acc gcc tat cgt     2449
   Phe Ser Arg Ser Pro Phe Tyr Asp Gly Asn Asp Pro Thr Ala Tyr Arg
                   395                 400                 405
   tgg tat tgc cag cac aaa cac gtg cgc cgg tta gct gat ctt ctt aag     2497
   Trp Tyr Cys Gln His Lys His Val Arg Arg Leu Ala Asp Leu Leu Lys
               410                 415                 420
   gag ata cgc act atg acg ggt caa gag cag tac acc aag cct cag caa     2545
   Glu Ile Arg Thr Met Thr Gly Gln Glu Gln Tyr Thr Lys Pro Gln Gln
           425                 430                 435
   cag ttt tct agt ggc cgc gag ctt att aat gct gtg aaa act cgc aga     2593
   Gln Phe Ser Ser Gly Arg Glu Leu Ile Asn Ala Val Lys Thr Arg Arg
       440                 445                 450
   gaa gaa gcc caa gca ttt aaa gca ctt gcc cag gac gtc gag acc gaa     2641
   Glu Glu Ala Gln Ala Phe Lys Ala Leu Ala Gln Asp Val Glu Thr Glu
   455                 460                 465                 470
   ctc agc aca gag ctt gat cag tac gac ccg gaa ctt atc gaa ggg gta     2689
   Leu Ser Thr Glu Leu Asp Gln Tyr Asp Pro Glu Leu Ile Glu Gly Val
                   475                 480                 485
   cga gtc tta tgg att agc cag ggg cgt gct gcc cgg gat gag acg gcg     2737
   Arg Val Leu Trp Ile Ser Gln Gly Arg Ala Ala Arg Asp Glu Thr Ala
               490                 495                 500
   ttt cgt tac gct ttg aaa acc tgc cac cgg cta cgg gcc gca ggt gag     2785
   Phe Arg Tyr Ala Leu Lys Thr Cys His Arg Leu Arg Ala Ala Gly Glu
           505                 510                 515
   cgt atg act gat gcc gcg atc att gat gcc tat gag cat gcg tat aac     2833
   Arg Met Thr Asp Ala Ala Ile Ile Asp Ala Tyr Glu His Ala Tyr Asn
       520                 525                 530
   gtt gct cag cgc cat ggg gga gac ggc cgg gat agt gag atg ccg ccg     2881
   Val Ala Gln Arg His Gly Gly Asp Gly Arg Asp Ser Glu Met Pro Pro
   535                 540                 545                 550
   atg cgg gat cgc cag acg atg gcg cgt cgc gtg cgc ggc tac gtg act     2929
   Met Arg Asp Arg Gln Thr Met Ala Arg Arg Val Arg Gly Tyr Val Thr
                   555                 560                 565
   caa tct aag acc agt atg ggc gca tca gcc cct cca ggg cgt gct aca     2977
   Gln Ser Lys Thr Ser Met Gly Ala Ser Ala Pro Pro Gly Arg Ala Thr
               570                 575                 580
   agc act gaa cgt aaa gca tta tcc acg atg ggg cgt cga ggc ggt aaa     3025
   Ser Thr Glu Arg Lys Ala Leu Ser Thr Met Gly Arg Arg Gly Gly Lys
           585                 590                 595
   aag gcc gca gaa cgc tgg aaa gac cgt gag agc cat tac gcg caa act     3073
   Lys Ala Ala Glu Arg Trp Lys Asp Arg Glu Ser His Tyr Ala Gln Thr
       600                 605                 610
   gaa ttg gaa aag ctt gcc gat gcc agt aag aag cgt tca aga aaa gcc    3121
   Glu Leu Glu Lys Leu Ala Asp Ala Ser Lys Lys Arg Ser Arg Lys Ala
   615                 620                 625                 630
   aaa ggc acg cgc tta act att gcg ggc tgg gtg atg agt gtg gaa tct    3169
   Lys Gly Thr Arg Leu Thr Ile Ala Gly Trp Val Met Ser Val Glu Ser
                   635                 640                 645
   gag aca ggt gca tgg cct act atc gct gag gcg atg gtg gag ttt tcg    3217
   Glu Thr Gly Ala Trp Pro Thr Ile Ala Glu Ala Met Val Glu Phe Ser
               650                 655                 660
   gtc tct aga gag act gta aaa agg gcg ctt aga tct gct gga att gag    3265
   Val Ser Arg Glu Thr Val Lys Arg Ala Leu Arg Ser Ala Gly Ile Glu
           665                 670                 675
   ctt cca cgg ggc aga cga aag acc tca aat taaatggctc acttcgtaag      3315
   Leu Pro Arg Gly Arg Arg Lys Thr Ser Asn
       680                 685
   caatatacgg ttccccgtgc acagcacggg ggggcttaac tcttgctctt ttaagcttta 3375
   attaaatagt tcaggttata agcaatatac ggttttcctg gtcttgtgca gggaggccac 3435
   tttacttcgg cctttgaaag tgaattgtgt ttcaaattaa aggtgcttct gaagaccttt 3495
   aatctctagg gagtttttct gtaggaggca gttgggtcta gccagggttg ataagtgatt 3555
   tcagtgagtg tccttctaga atgaaaagct tacgagtcgt ttaggcatat aacgggtgac 3615
   tagcgagttc agactttaaa agcgcaaaca aatttaatga ggtaatgcta tgagaaacgt 3675
   tcttttaact tgtccaatcc gtggcgagct tactgctact tcccttgctt ctgatgggct 3735
   tacgcctacc gaagaggcga tgaggattga tttgcttgag tttcttatag ataaacgtga 3795
   ctacccaaaa gattttattg atgttgaaac tgtggtgctg agcaatatcg gtaatgcagg 3855
   gcgcaatagt cttcgtgcgg acgtcattgt gtatgacatc ccgaagatgc aagcacgggc 3915
   catgtcacat gaagaacgac taattcatgc gacgttgatc gcagaggtga aacgagaggg 3975
   gaaatacaag aaaagcgctg tttctcatca gttagttccg gcattgaagc tcgctccatc 4035
   tatgaagaca cttggaattt actgggataa cgaggaaagg ctcctctttc agaagacttt 4095
   ttcagatgaa atgctttcgg ttgaagagat caccgttgca aaactcccaa agtggggttt 4155
   ttccctcaca ggaaaccccc tcacttacaa tcaactttcc tctccgaaag atttgttcaa 4215
   aactctaagt ggtgtcgctg acattatgcg gagtggtgga gtcgaagata aacaactgcg 4275
   ctatatcgaa acagtcaagt tgcttcttgc taggtataca gatgaacgca gtgcttctga 4335
   tccacaagat aaaaacggcg gagttcttgt gatgcagatt ttgtctgacg gtgaccctaa 4395
   ctttcgaaac cgaatggatg atctctataa gcgttcagcc gcgcgttaca gcaaagcgaa 4455
   gactctattc gcgaataaga cgtcacagct tgatgatgcc acgctccgtc aattagtggt 4515
   aaagattcaa ggttttcggt taac 4539
<210> 2
   <211> 199
   <212> ParA(pTET3)-PRT
   <213> Corynebacterium glutamicum LP-6
<400> 2
   Met Ser Ile Leu Thr Ile Ala His Thr Lys Gly Gly Val Gly Lys Thr
   1               5                   10                  15
   Thr Ser Ala Val Leu Leu Cys Ala Ala Ala His Ala Arg Gly Leu Ala
               20                  25                  30
   Val Thr Leu Ile Asp Ser Asp Ala Gln Gly Thr Ala Thr Ala Trp Ala
           35                  40                  45
   His Ala Ala Glu Glu Ala Gly Asp Thr Phe Pro Trp Pro Ile Ile Thr
       50                  55                  60
   Ala Ala Thr Pro Ala His Leu Ala Arg Thr Leu Asp Gly His Asn Gly
   65                  70                  75                  80
   Leu Val Ile Val Asp Thr Pro Pro Gly Gly Tyr Glu Val Ile Glu Thr
                   85                  90                  95
   Ala Ile Glu Ala Gly Asp Leu Ile Leu Ile Pro Thr Ser Ala Ser Pro
               100                 105                 110
   Leu Asp Ile Asn Arg Val Trp Pro Thr Val Glu Ala Thr Ser His Lys
           115                 120                 125
   Pro Ala Val Val Cys Leu Ser Gln Ile Asp Ser Arg Thr Thr Leu Pro
       130                 135                 140
   Lys Ile Ala Arg Thr Ala Leu Glu Asn Glu Gly Val Val Val Ala Glu
   145                 150                 155                 160
   Thr Glu Ile Pro Ala Arg Glu Ala Leu Arg His Met Tyr Ala Thr Thr
                   165                 170                 175
   Pro Gln Arg Leu Tyr Gly Tyr Asp Glu Leu Leu Thr Glu Leu Leu Ser
               180                 185                 190
   Ser Asn Leu Leu Gly Glu His
           195
<210> 3
   <211> 489
   <212> RepA(pTET3)-PRT
   <213> Corynebacterium glutamicum LP-6
<400> 3
   Met Ser Leu Pro Ser Lys Gly Arg Ser Ser Thr Pro Thr Gly Val Arg
    1               5                  10                  15
   Val Ala Gln Pro Leu Pro Thr His Arg Asp Thr Gly Gly Leu Asp Asp
               20                  25                  30
   Thr Pro Ala Gly Phe Thr Asp Arg Asp Ala Leu Ile Asp His Leu Gly
           35                  40                  45
   Arg Lys Ala Ile His Gly Ser Lys Asp Arg Asp Phe Gly Lys Ala Tyr
       50                  55                  60
   Tyr Arg His Glu Asp Gly Thr Leu Arg Pro Arg Leu Tyr Arg Val Asp
   65                  70                  75                  80
   Ser Glu Ala Leu Thr Arg Cys Gln Tyr Val Met Leu Thr Thr Gln Gln
                   85                  90                  95
   Tyr Ala Ala Val Leu Val Val Asp Ile Asp Gln Pro Gly Gln Ser Gly
               100                 105                 110
   Gly His Pro Ala Asn Leu Ser Pro Glu Val Arg Gln Lys Met Ala Ala
           115                 120                 125
   Leu Ile Glu His Asn Leu Gly Pro Ser Trp Val Gly Ile Asn Pro Gln
       130                 135                 140
   Asn Gly Lys Ala Gln Ala Ile Trp Leu Ile Asp Pro Val Tyr Ala Asp
   145                 150                 155                 160
   Lys Ser Gly Lys Ser Arg His Met Ser Leu Leu Ala Ala Thr Ser Arg
                   165                 170                 175
   Ala Leu Gly Glu Leu Leu Asp His Asp Pro Asn Phe Ser His Arg Phe
               180                 185                 190
   Ser Arg Ser Pro Phe Tyr Asp Gly Asn Asp Pro Thr Ala Tyr Arg Trp
           195                 200                 205
   Tyr Cys Gln His Lys His Val Arg Arg Leu Ala Asp Leu Leu Lys Glu
       210                 215                 220
   Ile Arg Thr Met Thr Gly Gln Glu Gln Tyr Thr Lys Pro Gln Gln Gln
   225                 230                 235                 240
   Phe Ser Ser Gly Arg Glu Leu Ile Asn Ala Val Lys Thr Arg Arg Glu
                   245                 250                 255
   Glu Ala Gln Ala Phe Lys Ala Leu Ala Gln Asp Val Glu Thr Glu Leu
               260                 265                 270
   Ser Thr Glu Leu Asp Gln Tyr Asp Pro Glu Leu Ile Glu Gly Val Arg
           275                 280                 285
   Val Leu Trp Ile Ser Gln Gly Arg Ala Ala Arg Asp Glu Thr Ala Phe
       290                 295                 300
   Arg Tyr Ala Leu Lys Thr Cys His Arg Leu Arg Ala Ala Gly Glu Arg
   305                 310                 315                 320
   Met Thr Asp Ala Ala Ile Ile Asp Ala Tyr Glu His Ala Tyr Asn Val
                   325                 330                 335
   Ala Gln Arg His Gly Gly Asp Gly Arg Asp Ser Glu Met Pro Pro Met
               340                 345                 350
   Arg Asp Arg Gln Thr Met Ala Arg Arg Val Arg Gly Tyr Val Thr Gln
           355                 360                 365
   Ser Lys Thr Ser Met Gly Ala Ser Ala Pro Pro Gly Arg Ala Thr Ser
       370                 375                 380
   Thr Glu Arg Lys Ala Leu Ser Thr Met Gly Arg Arg Gly Gly Lys Lys
   385                 390                 395                 400
   Ala Ala Glu Arg Trp Lys Asp Arg Glu Ser His Tyr Ala Gln Thr Glu
                   405                 410                 415
   Leu Glu Lys Leu Ala Asp Ala Ser Lys Lys Arg Ser Arg Lys Ala Lys
               420                 425                 430
   Gly Thr Arg Leu Thr Ile Ala Gly Trp Val Met Ser Val Glu Ser Glu
           435                 440                 445
   Thr Gly Ala Trp Pro Thr Ile Ala Glu Ala Met Val Glu Phe Ser Val
       450                 455                 460
   Ser Arg Glu Thr Val Lys Arg Ala Leu Arg Ser Ala Gly Ile Glu Leu
   465                 470                 475                 480
   Pro Arg Gly Arg Arg Lys Thr Ser Asn
                   485
<210> 4
   <211> 1856
   <212> DNA
   <213> Corynebacterium glutamicum LP-6
<220>
   <221> CDS
   <222> (338)..(1291)
   <223> repA
<400> 4
   gcatgccaat aaaagtcaac ccgccgtggc cacccaaaac cagagtgtaa ataaacattg 60
   agatagcttg atacctagac atctttccgc ctgataccta gacattaaga cgtctagatt 120
   gcttgctatc tacaacccct cacccaggga ctaaattaat attccaacaa agaaggactc 180
   ccatgttttc gtattctaaa tatcgaatac ctgatcggcg tttcgggcag ctatgtcaca 240
   tgtgactgct acgatcaggg gaaaattaaa agacctggca ctgccgcaaa cagtccaggt 300
   catgacccca cactatttct aagagacatg aggattt atg gat gat cat act ctg   355
                                            Met Asp Asp His Thr Leu
                                             1               5
   cca cag cca gat tcg ggc aac tca gat cca att agc gat ttg gaa gcg    403
   Pro Gln Pro Asp Ser Gly Asn Ser Asp Pro Ile Ser Asp Leu Glu Ala
               10                  15                  20
   cgc ctc gca gag atc gag gct ggc ctc ggc gat ccg ctc agt ttc acg    451
   Arg Leu Ala Glu Ile Glu Ala Gly Leu Gly Asp Pro Leu Ser Phe Thr
           25                  30                  35
   tca aaa acc ctg atc cag gca act ttt cca cac agt gca aaa gcc gga    499
   Ser Lys Thr Leu Ile Gln Ala Thr Phe Pro His Ser Ala Lys Ala Gly
       40                  45                  50
   aaa gaa ctt gtc ctg gtt aac ggc cat acg aca gtc acg atg tac agc    547
   Lys Glu Leu Val Leu Val Asn Gly His Thr Thr Val Thr Met Tyr Ser
   55                  60                  65                  70
   cgc cac ggc ctg cca tat ggc tca tgg cca cgg ctc att atg tgc tgg    595
   Arg His Gly Leu Pro Tyr Gly Ser Trp Pro Arg Leu Ile Met Cys Trp
                   75                  80                  85
   cta aca agg gaa gcc ctt cgc cgc caa aat ctc cca att gat gag gct    643
   Leu Thr Arg Glu Ala Leu Arg Arg Gln Asn Leu Pro Ile Asp Glu Ala
               90                  95                  100
   cgt gaa atc ccg ctt aat tca agc ttg agt ggt ttt atg cgg gaa gtt    691
   Arg Glu Ile Pro Leu Asn Ser Ser Leu Ser Gly Phe Met Arg Glu Val
           105                 110                 115
   ggc atc gga cgt gca acc gga ggg gag cgt ggc acg atc acc gcg ctg    739
   Gly Ile Gly Arg Ala Thr Gly Gly Glu Arg Gly Thr Ile Thr Ala Leu
       120                 125                 130
   aaa aag cag atg cgg tct ctt ttc tcc act tca atc ggc att gac atc    787
   Lys Lys Gln Met Arg Ser Leu Phe Ser Thr Ser Ile Gly Ile Asp Ile
   135                 140                 145                 150
   aaa gga gat gac gac ctt aag ctc ctg gat ctt gat gaa tca gtt atc    835
   Lys Gly Asp Asp Asp Leu Lys Leu Leu Asp Leu Asp Glu Ser Val Ile
                   155                 160                 165
   gct gag cgg acg gag atg tgg tgg acg ccg cga ccc cac gat gac atc    883
   Ala Glu Arg Thr Glu Met Trp Trp Thr Pro Arg Pro His Asp Asp Ile
               170                 175                 180
   gat ttt gag gga tat att cga ctc tcc gct act ttc tac tca gat ctc    931
   Asp Phe Glu Gly Tyr Ile Arg Leu Ser Ala Thr Phe Tyr Ser Asp Leu
           185                 190                 195
   atc aaa tca gcc gtc ccc ctc gac acc cga atc ctc cgc agt cta aag    979
   Ile Lys Ser Ala Val Pro Leu Asp Thr Arg Ile Leu Arg Ser Leu Lys
       200                 205                 210
   aaa tct ccg atg gcc atc gat gtc tac tct tgg ctc acc tac aga gtt    1027
   Lys Ser Pro Met Ala Ile Asp Val Tyr Ser Trp Leu Thr Tyr Arg Val
   215                 220                 225                 230
   tca tac ttg cgc tac ccc aca gta att aag tgg gat cag atc caa gga    1075
   Ser Tyr Leu Arg Tyr Pro Thr Val Ile Lys Trp Asp Gln Ile Gln Gly
                   235                 240                 245
   cag cta ggc gct ggc tac cct gac act tct caa gga atg cga aac ttc    1123
   Gln Leu Gly Ala Gly Tyr Pro Asp Thr Ser Gln Gly Met Arg Asn Phe
               250                 255                 260
   agg aag aaa ttt ttg atc gcc ctc aac aaa gtc att gac gta tgg ccc    1171
   Arg Lys Lys Phe Leu Ile Ala Leu Asn Lys Val Ile Asp Val Trp Pro
           265                 270                 275
   acc gac tcg atc agc atc gta aaa aac gga att cta ctg acc cct ggt    1219
   Thr Asp Ser Ile Ser Ile Val Lys Asn Gly Ile Leu Leu Thr Pro Gly
       280                 285                 290
   tca cca agc gtt ccc cgc aga gca cag gat gag ttc caa aaa cgc ttt    1267
   Ser Pro Ser Val Pro Arg Arg Ala Gln Asp Glu Phe Gln Lys Arg Phe
   295                 300                 305                 310
   tcg att ggt gat gat cca ctt ttt taaatcgata agtccccgca cttaggagtg  1321
   Ser Ile Gly Asp Asp Pro Leu Phe
                   315
   cggggatttt tcatgcccaa atacgtgcgc agtaacggta ccgcccgtgc gcagtaacgg 1381
   taccgcccgt gcgcagtaac ggtaccgccc gtgcgcagta acggtaccgc ccgtgcgcag 1441
   taacggtacc ggaacctatt atatattagc aggtcaaagt atgtttccaa ggtctcccct 1501
   ataggtcctt tagggcctat acaaccttta caactaccta tatgcaaaga aacttcaatt 1561
   catgttcggg tagcagaaaa ttgtccgaaa ctagcgttac acgaaatgca aatacgtatc 1621
   taagtatata actgaaatat aaaaacggca gaccgtaatt attaattaga aaacccgccc 1681
   tggaattatc caaagcggga ataaaagggt taagggaaac tagcgagcat tttctgattt 1741
   ctcggcatta ggaccgaccc acttccctct acgaccaaac tgttttgtgt cagagggttg 1801
   tgcacactca gtgtcatgac cttatgcaca ctcaatctca tgaccttgtg catgc      1856
<210> 5
   <211> 318
   <212> RepA(pCRY4)-PRT
   <213> Corynebacterium glutamicum LP-6
<400> 5
   Met Asp Asp His Thr Leu Pro Gln Pro Asp Ser Gly Asn Ser Asp Pro
    1               5                  10                  15
   Ile Ser Asp Leu Glu Ala Arg Leu Ala Glu Ile Glu Ala Gly Leu Gly
               20                  25                  30
   Asp Pro Leu Ser Phe Thr Ser Lys Thr Leu Ile Gln Ala Thr Phe Pro
           35                  40                  45
   His Ser Ala Lys Ala Gly Lys Glu Leu Val Leu Val Asn Gly His Thr
       50                  55                  60
   Thr Val Thr Met Tyr Ser Arg His Gly Leu Pro Tyr Gly Ser Trp Pro
   65                  70                  75                  80
   Arg Leu Ile Met Cys Trp Leu Thr Arg Glu Ala Leu Arg Arg Gln Asn
                   85                  90                  95
   Leu Pro Ile Asp Glu Ala Arg Glu Ile Pro Leu Asn Ser Ser Leu Ser
               100                 105                 110
   Gly Phe Met Arg Glu Val Gly Ile Gly Arg Ala Thr Gly Gly Glu Arg
           115                 120                 125
   Gly Thr Ile Thr Ala Leu Lys Lys Gln Met Arg Ser Leu Phe Ser Thr
       130                 135                 140
   Ser Ile Gly Ile Asp Ile Lys Gly Asp Asp Asp Leu Lys Leu Leu Asp
   145                 150                 155                 160
   Leu Asp Glu Ser Val Ile Ala Glu Arg Thr Glu Met Trp Trp Thr Pro
                   165                 170                 175
   Arg Pro His Asp Asp Ile Asp Phe Glu Gly Tyr Ile Arg Leu Ser Ala
               180                 185                 190
   Thr Phe Tyr Ser Asp Leu Ile Lys Ser Ala Val Pro Leu Asp Thr Arg
           195                 200                 205
   Ile Leu Arg Ser Leu Lys Lys Ser Pro Met Ala Ile Asp Val Tyr Ser
       210                 215                 220
   Trp Leu Thr Tyr Arg Val Ser Tyr Leu Arg Tyr Pro Thr Val Ile Lys
   225                 230                 235                 240
   Trp Asp Gln Ile Gln Gly Gln Leu Gly Ala Gly Tyr Pro Asp Thr Ser
                   245                 250                 255
   Gln Gly Met Arg Asn Phe Arg Lys Lys Phe Leu Ile Ala Leu Asn Lys
               260                 265                 270
   Val Ile Asp Val Trp Pro Thr Asp Ser Ile Ser Ile Val Lys Asn Gly
           275                 280                 285
   Ile Leu Leu Thr Pro Gly Ser Pro Ser Val Pro Arg Arg Ala Gln Asp
       290                 295                 300
   Glu Phe Gln Lys Arg Phe Ser Ile Gly Asp Asp Pro Leu Phe
   305                 310                 315
<210> 6
   <211> 7316
   <212> DNA
   <213> Corynebacterium glutamicum LP-6
<220>
   <221> gene
   <222> Complement ((1447)..(2013))
   <223> tetR
<220>
   <221> CDS
   <222> (2129)..(3272)
   <223> tetA
<220>
   <221> CDS
   <222> (5882)..(6718)
   <223> aadA
<400> 6
   aagcttgagc atgcttggcg gagattggac ggacggaacg atgacggatt tcaagtggcg 60
   ccatttccag ggtgatgtga tcctgtgggc ggtgcgctgg tattgtcgct atccgatcag 120
   ctatcgcgac cttgaggaaa tgctggcgga acgcggcatt tcggtcgacc atacgacgat 180
   ctatcgctgg gtccagtgct acgccccgga gatggagaag cggctgcgct ggttctggcg 240
   gcgtggcttt gatccgagct ggcgcctgga tgaaacctac gtcaaggtgc ggggcaagtg 300
   gacctacctg taccgggcag tcgacaagcg gggcgacacg atcgatttct acctgtcgcc 360
   gacccgcagc gccaaggcag cgaagcggtt cctgggcaag gccctgcgag gcctgaagca 420
   ctgggaaaag cctgccacgc tcaataccga caaagcgccg agctatggtg cagcgatcac 480
   cgaattgaag cgcgaaggaa agctggaccg ggagacggcc caccggcagg tgaagtatct 540
   caataacgtg atcgaggccg atcacggaaa gctcaagata ctgatcaagc cggtgcgcgg 600
   tttcaaatcg atccccacgg cctatgccac gatcaaggga ttcgaagtca tgcgagccct 660
   gcgcaaagga caggctcgcc cctggtgcct gcagcccggc atcaggggcg aggtgcgcct 720
   tgtggagaga gcttttggca ttgggccctc ggcgctgacg gaggccatgg gcatgctcaa 780
   ccaccatttc gcagcagccg cctgatcggc gcagagcgac agcctacctc tgactgccgc 840
   caatctttgc aacagagcct ttgcgtcaat gcagggagat agcgaagagc gcgcttcaac 900
   ggagatgctc gaatgggtcc acgacggatt ggagtccgtg gtcgcggcag acgtagatga 960
   ttcgcacgcc gtacccgtcg gcgccgctcg gctcggggtc gcattctgcg cggcagacgt 1020
   tacagagccg gtgctcgttg ctcccccaga ccgtgacctc gatatcgtcg gggatctcca 1080
   ttccgtcgaa ctccatatgc ggaggttagc tgtcgcggat tgagtcgtgt caagatgcgg 1140
   caccgatgct aaaccgccgt tacctatggt catcgcgccg gtcgcgcact cgacgcttag 1200
   ttcttgaggt actcgaggac ggcgatgacg cgcttgttcc ctgtgcgctc gttaaggtcg 1260
   agcatggtga agatgctgct gatgtgccgt tccgcgatcg cgacgcgaca tgcacacggt 1320
   ccctgatttg ctcgtttgtg agccccgtag ccatgagcga atcgtcagta tcgcggagga 1380
   ggtgctgcgg gagcgggaaa ggattgacct tactgacgca gagacccaaa gtgcgagcat 1440
   ccctcatcgc tttgatgcca gcccttcaac cattgcaact aacccgaact cgaaatctag 1500
   gtcttgatcg acaggctcac atccgttgtc gagcgctgtt tgttcttcta gtacgaaacc 1560
   gaccgtatag cggctgatag ccatgagagc tcggaccgca gagccctcag cgaatccttc 1620
   ggacacgaga aactcgatct gactttcggg ggcatccgag cccgctggca tctggtcact 1680
   cttttgacgg tgaaactctg cgtgcagccg tgctccatcc cggactgcca gaagcgctgt 1740
   ccggaagctc cgcgcgttgc gcaggagaaa gtcgtcccag cgctcccctg actctgggag 1800
   tgaggcgtgg tgttcgcgat caagcacatc agctgcgagc gatccgagca ggtgggcctt 1860
   tgtccgaaag tgccagtaga gcgctggctg ctgcacccgc agatgcgcag ccagcgcccg 1920
   tgtggtgaaa ccgtcgatcc ccgtgttatt gagcacatgc ctcgcaccgc gcaagactgc 1980
   tgcacgatcg agtcgcgctt gtttctgagc catgcttgca ctttatcatc gataacttta 2040
   tcgttgataa ggtgtcatct ctcacttccg ctcgtggctc gttggccacg gtcctcatca 2100
   cggctagcct cgacgccgcc ggc atg ggc ctg gtg atg ccg att ctt ccc gca 2153
                             Met Gly Leu Val Met Pro Ile Leu Pro Ala
                             1               5                   10
   ctg cta cac gag gca ggg gtc acc gct gat gcg gtt ccg ctg aac gtc    2201
   Leu Leu His Glu Ala Gly Val Thr Ala Asp Ala Val Pro Leu Asn Val
                   15                  20                  25
   gga gtg ctg atc gcg ctc tac gcg gta atg cag ttc atc ttt gcc ccc    2249
   Gly Val Leu Ile Ala Leu Tyr Ala Val Met Gln Phe Ile Phe Ala Pro
               30                  35                  40
   gta ctg gga acg ctg tcg gac cga ttc ggc cgc cgc cgg gtg ctg ctt    2297
   Val Leu Gly Thr Leu Ser Asp Arg Phe Gly Arg Arg Arg Val Leu Leu
           45                  50                  55
   gtt tcc ctg gcc ggt gcg acc gtc gac tat ctc gtg ctc gcc acg acg    2345
   Val Ser Leu Ala Gly Ala Thr Val Asp Tyr Leu Val Leu Ala Thr Thr
       60                  65                  70
   tcc gct ctg tcg gtg ttc tat atc gcc cgc gca gtg gct ggg ata acc    2393
   Ser Ala Leu Ser Val Phe Tyr Ile Ala Arg Ala Val Ala Gly Ile Thr
   75                  80                  85                  90
   gga gcg acc aat gcg gtc acc gcc acc gtg atc gcc gac atc acg cca    2441
   Gly Ala Thr Asn Ala Val Thr Ala Thr Val Ile Ala Asp Ile Thr Pro
                   95                  100                 105
   ccc cac cag cgc gcc aag cgt ttc ggt tta ctc agt gcc tgc tat ggc    2489
   Pro His Gln Arg Ala Lys Arg Phe Gly Leu Leu Ser Ala Cys Tyr Gly
               110                 115                 120
   ggc gga atg atc gcg ggg cca gcc atg ggt gga ctg ttc ggt gcc atc    2537
   Gly Gly Met Ile Ala Gly Pro Ala Met Gly Gly Leu Phe Gly Ala Ile
           125                 130                 135
   tcg cca cat ctg ccg ttt ttg ctc gct gct ctt ctc tca gcg agc aat    2585
   Ser Pro His Leu Pro Phe Leu Leu Ala Ala Leu Leu Ser Ala Ser Asn
       140                 145                 150
   ctg gca ctc acc ttt atc ctg tta cgc gag acc cgt cct gat tcc cct    2633
   Leu Ala Leu Thr Phe Ile Leu Leu Arg Glu Thr Arg Pro Asp Ser Pro
   155                 160                 165                 170
   gcg cgc tct gcg tcg ctc gct cag cat cgt ggt cgc ccc ggc ctc agc    2681
   Ala Arg Ser Ala Ser Leu Ala Gln His Arg Gly Arg Pro Gly Leu Ser
                   175                 180                 185
   gcg gtg cct ggg att acc ttc cta tta atc gca ttc ggc ctt gtt caa    2729
   Ala Val Pro Gly Ile Thr Phe Leu Leu Ile Ala Phe Gly Leu Val Gln
               190                 195                 200
   ttc att ggg cag gct cca ggt gcg acc tgg gtg ctg ttt act gaa cac    2777
   Phe Ile Gly Gln Ala Pro Gly Ala Thr Trp Val Leu Phe Thr Glu His
           205                 210                 215
   cgc ctc gac tgg agt ccc gtc gaa gtt gga atc tcc ctg tcc gtt ttc    2825
   Arg Leu Asp Trp Ser Pro Val Glu Val Gly Ile Ser Leu Ser Val Phe
       220                 225                 230
   ggg atc gta cag gtt ctc gtg cag gcc ctc ctt act ggc cgc atc gtg    2873
   Gly Ile Val Gln Val Leu Val Gln Ala Leu Leu Thr Gly Arg Ile Val
   235                 240                 245                 250
   gag tgg atc ggt gag gca aaa aca gtc atc atc ggg tgt att acc gac    2921
   Glu Trp Ile Gly Glu Ala Lys Thr Val Ile Ile Gly Cys Ile Thr Asp
                   255                 260                 265
   gcc ttg ggt ctc gta ggc ctg gcg att gtc act gac gca ttt tcc atg    2969
   Ala Leu Gly Leu Val Gly Leu Ala Ile Val Thr Asp Ala Phe Ser Met
               270                 275                 280
   gca cct atc ttg gcg gca ctg ggg atc ggt ggc atc ggc ctc ccc gct    3017
   Ala Pro Ile Leu Ala Ala Leu Gly Ile Gly Gly Ile Gly Leu Pro Ala
           285                 290                 295
   ctg caa acc ctt ctc tcc cag cgc gtc gat gaa cag cac caa ggg cgc    3065
   Leu Gln Thr Leu Leu Ser Gln Arg Val Asp Glu Gln His Gln Gly Arg
       300                 305                 310
   ctc cag ggt gtg ctc gcc agc atc aac agc gtc aca tcg atc ttc gga    3113
   Leu Gln Gly Val Leu Ala Ser Ile Asn Ser Val Thr Ser Ile Phe Gly
   315                 320                 325                 330
   ccg gtc gct ttc aca acg atc ttc gcg ctc act tac atc aac gcc gac    3161
   Pro Val Ala Phe Thr Thr Ile Phe Ala Leu Thr Tyr Ile Asn Ala Asp
                   335                 340                 345
   ggc ttc ctc tgg ctc tgc gcc gca gca ctc tac gtg ccc tgc gtg att    3209
   Gly Phe Leu Trp Leu Cys Ala Ala Ala Leu Tyr Val Pro Cys Val Ile
               350                 355                 360
   ctc atc atg cgt ggt aca gca gcg tcc ccg aag ttc ggc tct tgg gcg    3257
   Leu Ile Met Arg Gly Thr Ala Ala Ser Pro Lys Phe Gly Ser Trp Ala
           365                 370                 375
   agc ggc gac tcg atg tgagttgtga gacgtgagca ggagcaacac ggcggcgaca    3312
   Ser Gly Asp Ser Met
       380
   ctgcttcgcc atggccgact agcgagacgg cgccaccggg aaactcggca tcatctacca 3372
   aggacaggtc agctgggagc ctgatagacc catcgaaatg tgcgtgccga tcgcggagaa 3432
   gggccgggcg catcggatcg agccatagca ccatgagtct tcacggaagt gcgtcgacgg 3492
   agacttggtt gtgaaccggg ccaagggaga gctggaggcc ctctccgagt ggcttgccga 3552
   tgacatgagc tggacgctca tcgagaaatc cacacacagc ggccccagtg cagcccgaga 3612
   ggtgcgcccg ccgttctccc gagcgggtgg aggtcatttc tgtcgtcacc cacggacgac 3672
   gcgcttcctg cgacggctac ctcgaggctg gaggaatgcg cgtccgtttc agccatgcgt 3732
   tccgcttcgt cagcaccccc aagacctcga tgatcgcaga actgcgacgc tactgcatcg 3792
   agacgcaggt tgactgaggc ctgtgcggac agcacgaacg acccttgagc ccgtaatctg 3852
   ggaaccgcag aaactacccg atcgaaacgc aactactttg ccgaccctac ggggttggct 3912
   cgcggtcgtc gtccttggcc gggctctgtt gcaaaaatcg tgaagcttga gcatgcttgg 3972
   cggagattgg acggacggaa cgatgacgga tttcaagtgg cgccatttcc agggtgatgt 4032
   gatcctgtgg gcggtgcgct ggtattgtcg ctatccgatc agctatcgcg accttgagga 4092
   aatgctggcg gaacgcggca tttcggtcga ccatacgacg atctatcgct gggtccagtg 4152
   ctacgccccg gagatggaga agcggctgcg ctggttctgg cggcgtggct ttgatccgag 4212
   ctggcgcctg gatgaaacct acgtcaaggt gcggggcaag tggacctacc tgtaccgggc 4272
   agtcgacaag cggggcgaca cgatcgattt ctacctgtcg ccgacccgca gcgccaaggc 4332
   agcgaagcgg ttcctgggca aggccctgcg aggcctgaag cactgggaaa agcctgccac 4392
   gctcaatacc gacaaagcgc cgagctatgg tgcagcgatc accgaattga agcgcgaagg 4452
   aaagctggac cgggagacgg cccaccggca ggtgaagtat ctcaataacg tgatcgaggc 4512
   cgatcacgga aagctcaaga tactgatcaa gccggtgcgc ggtttcaaat cgatccccac 4572
   ggcctatgcc acgatcaagg gattcgaagt catgcgagcc ctgcgcaaag gacaggctcg 4632
   cccctggtgc ctgcagcccg gcatcagggg cgaggtgcgc cttgtggaga gagcttttgg 4692
   cattgggccc tcggcgctga cggaggccat gggcatgctc aaccaccatt tcgcagcagc 4752
   cgcctgatcg gcgcagagcg acagcctacc tctgactgcc gccaatcttt gcaacagagc 4812
   cgtcgtagag acgtcggaat ggccgagcag atcctgcacg gttcgaatgt cgtaaccgct 4872
   gcggagcaag gccgtcgcga acgagtggcg gagggtgtgc ggtgtggcgg gcttcgtgat 4932
   gcctgcttgt tctacggcac gtttgaaggc gcgctgaaag gtctggtcat acatgtgatg 4992
   gcgacgcacg acaccgctcc gtggatcggt cgaatgcgtg tgctgcgcaa aaacccagaa 5052
   ccacggccag gaatgcccgg cgcgcggata cttccgctca agggcgtcgg gaagcgcaac 5112
   gccgctgcgg ccctcggcct ggtccttcag ccaccatgcc cgtgcacgcg acagctgctc 5172
   gcgcaggctg ggtgccaagc tctcgggtaa catcaaggcc cgatccttgg agcccttgcc 5232
   ctcccgcacg atgatcgtgc cgtgatcgaa atccagatcc ttgacccgca gttgcaaacc 5292
   ctcactgatc cgcatgcccg ttccatacag aagctgggcg aacaaacgat gctcgccttc 5352
   cagaaaaccg aggatgcgaa ccacttcatc cggggtcagc accaccggca agcgccgcga 5412
   cggccgaggt cttccgatct cctgaagcca gggcagatcc gtgcacagca ccttgccgta 5472
   gaagaacagc aaggccgcca atgcctgacg atgcgtggag accgaaacct tgcgctcgtt 5532
   cgccagccag gacagaaatg cctcgacttc gctgctgccc aaggttgccg ggtgacgcac 5592
   accgtggaaa cggatgaagg cacgaaccca gtggacatac gcctgttcgg ttcgtaagct 5652
   ataatgcaag tagcgtatgc gctcacgcaa ctggtccaga accttgaccg aacgcagcgg 5712
   tggtaacggt gcagtgctgg ttttcatggc ttgttatgac tgttttgttg tacagtctat 5772
   gcctcgggca tccaagcagc aagcgcgtta cgccgtgggt cgatgtttga tgttatggag 5832
   cagcaacgat gttacgcagc agggcagtcg ccctaaaaca aagttagac atg atg agc 5890
                                                        Met Met Ser
                                                            385
   aac tct ata cac acc gga atc tca aga cag ctt tca cag gca cgc gat   5938
   Asn Ser Ile His Thr Gly Ile Ser Arg Gln Leu Ser Gln Ala Arg Asp
               390                 395                 400
   gta att aaa cgc cat ttg gca tca acg ctg aaa gcc ata cac ttg tat   5986
   Val Ile Lys Arg His Leu Ala Ser Thr Leu Lys Ala Ile His Leu Tyr
           405                 410                 415
   ggt tct gca att gat ggt ggc ctc aaa cca tat agc gac att gat ctg   6034
   Gly Ser Ala Ile Asp Gly Gly Leu Lys Pro Tyr Ser Asp Ile Asp Leu
       420                 425                 430
   ctg gtt acc gtg gat gca cgc ttg gat gaa gct acc aga cgc tcc ctg    6082
   Leu Val Thr Val Asp Ala Arg Leu Asp Glu Ala Thr Arg Arg Ser Leu
   435                 440                 445                 450
   atg ctc gat ttc ttg aat atc tcg gca cca cca tgc gaa agc tca ata    6130
   Met Leu Asp Phe Leu Asn Ile Ser Ala Pro Pro Cys Glu Ser Ser Ile
                   455                 460                 465
   ctc cgg ccg cta gag gta act gtt gtt gca tgc aac gaa gta gtg cct    6178
   Leu Arg Pro Leu Glu Val Thr Val Val Ala Cys Asn Glu Val Val Pro
               470                 475                 480
   tgg cgt tat ccg gca cga cga gaa ctg cag ttc ggg gag tgg ctg cgg    6226
   Trp Arg Tyr Pro Ala Arg Arg Glu Leu Gln Phe Gly Glu Trp Leu Arg
           485                 490                 495
   gag gat att ctt gaa ggt gtc ttc gag cca gcc gcc ttg gac gcc gac    6274
   Glu Asp Ile Leu Glu Gly Val Phe Glu Pro Ala Ala Leu Asp Ala Asp
       500                 505                 510
   ctt gca att cta ata acg aaa gct agg caa cac agc atc gct tta gta    6322
   Leu Ala Ile Leu Ile Thr Lys Ala Arg Gln His Ser Ile Ala Leu Val
   515                 520                 525                 530
   ggt cca gtg gct caa aaa gtc ttc atg ccg gtg cca gag cat gac ttt    6370
   Gly Pro Val Ala Gln Lys Val Phe Met Pro Val Pro Glu His Asp Phe
                   535                 540                 545
   ctc cag gtg ctt tcc gat acc ctt aag ctg tgg aat act cat gag gat    6418
   Leu Gln Val Leu Ser Asp Thr Leu Lys Leu Trp Asn Thr His Glu Asp
               550                 555                 560
   tgg gaa aat gag gag cgg aac atc gta ctc acg tta gct cgg atc tgg    6466
   Trp Glu Asn Glu Glu Arg Asn Ile Val Leu Thr Leu Ala Arg Ile Trp
           565                 570                 575
   tat agc act gaa act gga gga atc gtc ccc aag gat gtg gcc gcc gaa    6514
   Tyr Ser Thr Glu Thr Gly Gly Ile Val Pro Lys Asp Val Ala Ala Glu
       580                 585                 590
   tgg gtt tta gag cgc ttg cca gct gag cat aag cca ata ctg gtt gag    6562
   Trp Val Leu Glu Arg Leu Pro Ala Glu His Lys Pro Ile Leu Val Glu
   595                 600                 605                 610
   gcg cgg caa gcc tat ctt ggg ctt tgc aag gat agt ctt gct ttg cgt    6610
   Ala Arg Gln Ala Tyr Leu Gly Leu Cys Lys Asp Ser Leu Ala Leu Arg
                   615                 620                 625
   gca gat gag act tcg gcg ttc att ggc tat gca aag tct gcg gtc gct    6658
   Ala Asp Glu Thr Ser Ala Phe Ile Gly Tyr Ala Lys Ser Ala Val Ala
               630                 635                 640
   gat ttg ctc gaa aag cga aaa tct caa act tcg cat att tgc gat ggc    6706
   Asp Leu Leu Glu Lys Arg Lys Ser Gln Thr Ser His Ile Cys Asp Gly
           645                 650                 655
   gcc aag aac gtc taacgtctaa ctattcattt aagccgaagc cgcttcgcgg      6758
   Ala Lys Asn Val
       660
   ctcggcttaa ttcaggcgtt agatgcacta agcacataat tgctcacagc caaactatca 6818
   ggtcaagtct gcttttatta tttttaagcg tgcataataa gccctacaca aattgggaga 6878
   tatatcatga aaggctggct ttttcttgtt atcgcaatag ttggcgaagt aatcgcaaca 6938
   tccgcattaa aatctagcga gggctttact aagcttgccc cttccgccgt tgtcataatc 6998
   ggttatggca tcgcatttta ttttctttct ctggttctga aatccatccc tgtcggtgtt 7058
   gcttatgcag tctggtcggg actcggcgtc gtcataatta cagccattgc ctggttgctt 7118
   catgggcaaa agcttgatgc gtggggcttt gtaggtatgg ggctcataat tgctgccttt 7178
   ttgctcgccc gatccccatc gtggaagtcg ctgcggaggc cgacgccatg gtgacggtgt 7238
   tcggcattct gaatctcacc gaggactcct tcttcgatga gagccggcgg ctagaccccg 7298
   ccggcgctgt caccgcgg                                               7316
<210> 7
   <211> 383
   <212> TetA-PRT
   <213> Corynebacterium glutamicum LP-6
<400> 7
   Met Gly Leu Val Met Pro Ile Leu Pro Ala Leu Leu His Glu Ala Gly
    1               5                  10                  15
   Val Thr Ala Asp Ala Val Pro Leu Asn Val Gly Val Leu Ile Ala Leu
               20                  25                  30
   Tyr Ala Val Met Gln Phe Ile Phe Ala Pro Val Leu Gly Thr Leu Ser
           35                  40                  45
   Asp Arg Phe Gly Arg Arg Arg Val Leu Leu Val Ser Leu Ala Gly Ala
       50                  55                  60
   Thr Val Asp Tyr Leu Val Leu Ala Thr Thr Ser Ala Leu Ser Val Phe
   65                  70                  75                  80
   Tyr Ile Ala Arg Ala Val Ala Gly Ile Thr Gly Ala Thr Asn Ala Val
                   85                  90                  95
   Thr Ala Thr Val Ile Ala Asp Ile Thr Pro Pro His Gln Arg Ala Lys
               100                 105                 110
   Arg Phe Gly Leu Leu Ser Ala Cys Tyr Gly Gly Gly Met Ile Ala Gly
           115                 120                 125
   Pro Ala Met Gly Gly Leu Phe Gly Ala Ile Ser Pro His Leu Pro Phe
       130                 135                 140
   Leu Leu Ala Ala Leu Leu Ser Ala Ser Asn Leu Ala Leu Thr Phe Ile
   145                 150                 155                 160
   Leu Leu Arg Glu Thr Arg Pro Asp Ser Pro Ala Arg Ser Ala Ser Leu
                   165                 170                 175
   Ala Gln His Arg Gly Arg Pro Gly Leu Ser Ala Val Pro Gly Ile Thr
               180                 185                 190
   Phe Leu Leu Ile Ala Phe Gly Leu Val Gln Phe Ile Gly Gln Ala Pro
           195                 200                 205
   Gly Ala Thr Trp Val Leu Phe Thr Glu His Arg Leu Asp Trp Ser Pro
       210                 215                 220
   Val Glu Val Gly Ile Ser Leu Ser Val Phe Gly Ile Val Gln Val Leu
   225                 230                 235                 240
   Val Gln Ala Leu Leu Thr Gly Arg Ile Val Glu Trp Ile Gly Glu Ala
                   245                 250                 255
   Lys Thr Val Ile Ile Gly Cys Ile Thr Asp Ala Leu Gly Leu Val Gly
               260                 265                 270
   Leu Ala Ile Val Thr Asp Ala Phe Ser Met Ala Pro Ile Leu Ala Ala
           275                 280                 285
   Leu Gly Ile Gly Gly Ile Gly Leu Pro Ala Leu Gln Thr Leu Leu Ser
       290                 295                 300
   Gln Arg Val Asp Glu Gln His Gln Gly Arg Leu Gln Gly Val Leu Ala
   305                 310                 315                 320
   Ser Ile Asn Ser Val Thr Ser Ile Phe Gly Pro Val Ala Phe Thr Thr
                   325                 330                 335
   Ile Phe Ala Leu Thr Tyr Ile Asn Ala Asp Gly Phe Leu Trp Leu Cys
               340                 345                 350
   Ala Ala Ala Leu Tyr Val Pro Cys Val Ile Leu Ile Met Arg Gly Thr
           355                 360                 365
   Ala Ala Ser Pro Lys Phe Gly Ser Trp Ala Ser Gly Asp Ser Met
       370                 375                 380
<210> 8
   <211> 279
   <212> AadA-PRT
   <213> Corynebacterium glutamicum LP-6
<400> 8
   Met Met Ser Asn Ser Ile His Thr Gly Ile Ser Arg Gln Leu Ser Gln
   1               5                   10                  15
   Ala Arg Asp Val Ile Lys Arg His Leu Ala Ser Thr Leu Lys Ala Ile
               20                  25                  30
   His Leu Tyr Gly Ser Ala Ile Asp Gly Gly Leu Lys Pro Tyr Ser Asp
           35                  40                      45
   Ile Asp Leu Leu Val Thr Val Asp Ala Arg Leu Asp Glu Ala Thr Arg
       50                  55                      60
   Arg Ser Leu Met Leu Asp Phe Leu Asn Ile Ser Ala Pro Pro Cys Glu
   65                  70                      75                  80
   Ser Ser Ile Leu Arg Pro Leu Glu Val Thr Val Val Ala Cys Asn Glu
                   85                      90                  95
   Val Val Pro Trp Arg Tyr Pro Ala Arg Arg Glu Leu Gln Phe Gly Glu
               100                     105                 110
   Trp Leu Arg Glu Asp Ile Leu Glu Gly Val Phe Glu Pro Ala Ala Leu
           115                 120                     125
   Asp Ala Asp Leu Ala Ile Leu Ile Thr Lys Ala Arg Gln His Ser Ile
       130                 135                     140
   Ala Leu Val Gly Pro Val Ala Gln Lys Val Phe Met Pro Val Pro Glu
   145                 150                     155                 160
   His Asp Phe Leu Gln Val Leu Ser Asp Thr Leu Lys Leu Trp Asn Thr
                   165                     170                 175
   His Glu Asp Trp Glu Asn Glu Glu Arg Asn Ile Val Leu Thr Leu Ala
               180                 185                     190
   Arg Ile Trp Tyr Ser Thr Glu Thr Gly Gly Ile Val Pro Lys Asp Val
           195                 200                     205
   Ala Ala Glu Trp Val Leu Glu Arg Leu Pro Ala Glu His Lys Pro Ile
       210                 215                     220
   Leu Val Glu Ala Arg Gln Ala Tyr Leu Gly Leu Cys Lys Asp Ser Leu
   225                 230                     235                 240
   Ala Leu Arg Ala Asp Glu Thr Ser Ala Phe Ile Gly Tyr Ala Lys Ser
                   245                     250                 255
   Ala Val Ala Asp Leu Leu Glu Lys Arg Lys Ser Gln Thr Ser His Ile
               260                 265                     270
   Cys Asp Gly Ala Lys Asn Val
           275
<210> 9
   <211> 570
   <212> DNA
   <213> Corynebacterium glutamicum LP-6
<220>
   <221> CDS
   <222> (1)..(567)
   <223> tetR
<400> 9
   atg gct cag aaa caa gcg cga ctc gat cgt gca gca gtc ttg cgc ggt      48
   Met Ala Gln Lys Gln Ala Arg Leu Asp Arg Ala Ala Val Leu Arg Gly
    1               5                  10                  15
   gcg agg cat gtg ctc aat aac acg ggg atc gac ggt ttc acc aca cgg      96
   Ala Arg His Val Leu Asn Asn Thr Gly Ile Asp Gly Phe Thr Thr Arg
               20                  25                  30
   gcg ctg gct gcg cat ctg cgg gtg cag cag cca gcg ctc tac tgg cac     144
   Ala Leu Ala Ala His Leu Arg Val Gln Gln Pro Ala Leu Tyr Trp His
           35                  40                  45
   ttt cgg aca aag gcc cac ctg ctc gga tcg ctc gca gct gat gtg ctt     192
   Phe Arg Thr Lys Ala His Leu Leu Gly Ser Leu Ala Ala Asp Val Leu
       50                  55                  60
   gat cgc gaa cac cac gcc tca ctc cca gag tca ggg gag cgc tgg gac     240
   Asp Arg Glu His His Ala Ser Leu Pro Glu Ser Gly Glu Arg Trp Asp
   65                  70                  75                  80
   gac ttt ctc ctg cgc aac gcg cgg agc ttc cgg aca gcg ctt ctg gca     288
   Asp Phe Leu Leu Arg Asn Ala Arg Ser Phe Arg Thr Ala Leu Leu Ala
                   85                  90                  95
   gtc cgg gat gga gca cgg ctg cac gca gag ttt cac cgt caa aag agt     336
   Val Arg Asp Gly Ala Arg Leu His Ala Glu Phe His Arg Gln Lys Ser
               100                 105                 110
   gac cag atg cca gcg ggc tcg gat gcc ccc gaa agt cag atc gag ttt     384
   Asp Gln Met Pro Ala Gly Ser Asp Ala Pro Glu Ser Gln Ile Glu Phe
           115                 120                 125
   ctc gtg tcc gaa gga ttc gct gag ggc tct gcg gtc cga gct ctc atg     432
   Leu Val Ser Glu Gly Phe Ala Glu Gly Ser Ala Val Arg Ala Leu Met
       130                 135                 140
   gct atc agc cgc tat acg gtc ggt ttc gta cta gaa gaa caa aca gcg     480
   Ala Ile Ser Arg Tyr Thr Val Gly Phe Val Leu Glu Glu Gln Thr Ala
   145                 150                 155                 160
   ctc gac aac gga tgt gag cct gtc gat caa gac cta gat ttc gag ttc     528
   Leu Asp Asn Gly Cys Glu Pro Val Asp Gln Asp Leu Asp Phe Glu Phe
                   165                 170                 175
   ggg tta gtt gca atg gtt gaa ggg ctg gca tca aag cga tga             570
   Gly Leu Val Ala Met Val Glu Gly Leu Ala Ser Lys Arg
               180                 185
<210> 10
   <211> 189
   <212> TetR-PRT
   <213> Corynebacterium glutamicum LP-6
<400> 10
   Met Ala Gln Lys Gln Ala Arg Leu Asp Arg Ala Ala Val Leu Arg Gly
    1               5                  10                  15
   Ala Arg His Val Leu Asn Asn Thr Gly Ile Asp Gly Phe Thr Thr Arg
               20                  25                  30
   Ala Leu Ala Ala His Leu Arg Val Gln Gln Pro Ala Leu Tyr Trp His
           35                  40                  45
   Phe Arg Thr Lys Ala His Leu Leu Gly Ser Leu Ala Ala Asp Val Leu
       50                  55                  60
   Asp Arg Glu His His Ala Ser Leu Pro Glu Ser Gly Glu Arg Trp Asp
   65                  70                  75                  80
   Asp Phe Leu Leu Arg Asn Ala Arg Ser Phe Arg Thr Ala Leu Leu Ala
                   85                  90                  95
   Val Arg Asp Gly Ala Arg Leu His Ala Glu Phe His Arg Gln Lys Ser
               100                 105                 110
   Asp Gln Met Pro Ala Gly Ser Asp Ala Pro Glu Ser Gln Ile Glu Phe
           115                 120                 125
   Leu Val Ser Glu Gly Phe Ala Glu Gly Ser Ala Val Arg Ala Leu Met
       130                 135                 140
   Ala Ile Ser Arg Tyr Thr Val Gly Phe Val Leu Glu Glu Gln Thr Ala
   145                 150                 155                 160
   Leu Asp Asn Gly Cys Glu Pro Val Asp Gln Asp Leu Asp Phe Glu Phe
                   165                 170                 175
   Gly Leu Val Ala Met Val Glu Gly Leu Ala Ser Lys Arg
               180                 185

## Beschreibung

Gegenstand der Erfindung sind die neuen Plasmide pTET3 und pCRY4 und deren Verwendung zur Herstellung von Vektorplasmiden.

### Stand der Technik

Natürlich vorkommende Plasmide und aus diesen hergestellte Plasmidvektoren sind eine wichtige Voraussetzung für die Verbesserung der Produktionseigenschaften von coryneformen Bakterien. Die Konstruktion von Plasmidvektoren für diese Gruppe industriell bedeutender Bakterien basiert im wesentlichen auf kryptischen Plasmiden, die mit geeigneten, in Corynebakterien oder Brevibakterien funktionsfähigen Selektionsmarkern ausgestattet werden (US-A 5,158,891 und US-A 4,500,640). Diese Plasmidvektoren können zur Klonierung und Verstärkung von Genen, die an der Produktion von L-Aminosäuren, Vitaminen oder Nukleotiden beteiligt sind, eingesetzt werden. Durch die Expression der jeweiligen Gene kann die Produktion der gewünschten Stoffe positiv beeinflußt werden. So führte z. B. die Klonierung eines DNA-Fragmentes, das ein Protein für einen Lysin-Exporter kodiert, zu einer Verbesserung der fermentativen Produktion von L-Lysin mit Corynebacterium glutamicum Stamm MH20-22B (DE-A 19548222).

Im Gegensatz zu dem bekannten und industriell gleichermaßen bedeutsamen Bakterium Escherichia coli steht für Corynebakterien und Brevibakterien nur eine begrenzte Anzahl von natürlichen Plasmiden und geeigneten Selektionsmarkern für die Entwicklung von Klonier- und Expressionsvektoren zur Verfügung. Viele der bekannten und mit einem gesonderten Namen versehenen Plasmide erwiesen sich bei näherer Analyse ihrer genetischen Organisation als identisch. Diese Plasmidisolate wurden daher in zwei Plasmidgruppen zusammengefaßt (Sonnen et al., Gene 107, 69-74 (1991)).

Zur pBL1-Gruppe gehören die Plasmide pAM286 aus Corynebacterium glutamicum AJ11560 (EP-A 0093611), pAM330 aus Brevibacterium lactofermentum ATCC13869 (Miwa et al., Agricultural and Biological Chemistry 48, 2901-2903 (1984)), pX18 aus Brevibacterium lactofermentum ATCC21086 (Yeh et al, Gene 47, 301-308 (1986)) und pBL1 aus Brevibacterium lactofermentum ATCC21798 (Santamaria et al., Journal of General Microbiology 130, 2237-2246 (1984)).

Die pHM1519-Gruppe umfaßt die Plasmide pCG1 aus Corynebacterium glutamicum ATCC31808 (US-A 4,617,267), pHM1519 aus Corynebacterium glutamicum ATCC13058 (Miwa et al., Agricultural and Biological Chemistry 48, 2901-2903 (1984)), pSR1 aus Corynebacterium glutamicum ATCC19223 (Yoshihama et al., Journal of Bacteriology 162, 591-597 (1985)) und pRN3.1 aus Corynebacterium glutamicum ATCC39269 (US-A 4,559,308).

Neben Mitgliedern dieser beiden Plasmidgruppen wurden noch die kryptischen Plasmide pCG2 aus Corynebacterium glutamicum ATCC31832 (US-A 4,489,160) und pAG3 aus Corynebacterium melassecola 22220 (US-A 5,158,891) isoliert.

Selektionssysteme stehen bislang lediglich in Form von zwei Antibiotikum-Resistenzmarkern zur Verfügung, die auf dem Streptomycin-/Spectinomycin-Resistenzplasmid pCG4 aus Corynebacterium glutamicum ATCC31830 (US-A 4,489,160) und auf dem Tetracyclin-Resistenzplasmid pAG1 aus Corynebacterium melassecola 22243 (US-A 5,158,891) identifiziert wurden. Plasmid pCG4 trägt außerdem das Sulfamethoxazolresistenz vermittelnde sulI-Gen, dessen Sequenz von Nesvera et al. (FEMS Microbiology Letters 169, 391-395 (1998)) bestimmt wurde.

Für die schnelle Untersuchung und Verbesserung von Stämmen, die Aminosäuren, Vitamine oder Nukleotide produzieren, ist es wichtig, Plasmidvektoren zu besitzen, die miteinander kompatibel sind und eine ausreichend hohe Stabiltät besitzen.

Aus dem Stand der Technik ist bekannt, daß pHM1519-Plasmidderivate und pBL1-Plasmidderivate koexistieren können. Außerdem ist bekannt, daß die in US-A 5,175,108 beschriebenen Plasmide pGA1 und pGA2 kompatibel sind.

Weiterhin sind Plasmidvektoren von Bedeutung, die hohe, mittlere oder niedrige Kopienzahlen aufweisen, um so eine abgestufte Expression des interessierenden Gens einstellen zu können. Die meisten der bekannten Plasmide besitzen eine hohe Kopienzahl. Lediglich von dem in der US-A 5,175,108 beschriebenen Plasmid pGA2 ist eine niedrige Kopienzahl bekannt.

Die landläufig eingesetzten Plasmidvektoren sind zusammengesetzt aus Komponenten, die aus der Spezies C. glutamicum und Komponenten einer anderen Bakterienspezies, typischerweise E. coli stammen. Durch diese Vorgehensweise wird artfremde DNA in die Spezies C. glutamicum eingeführt. Funktionsfähige Plasmidvektoren mit abgestufter Kopienzahl, die nur arteigene DNA enthalten und somit Kriterien der Selbstklonierung entsprechen sind in der Fachwelt nicht bekannt.

### Aufgabe der Erfindung

Die Erfinder haben sich zur Aufgabe gestellt, neue Plasmide, die für die Konstruktion von Plasmidvektoren mit verbesserten Eigenschaften geeignet sind, für Aminosäure-, Vitamin- und Nukleotid-produzierende coryneforme Bakterien bereitzustellen.

### Beschreibung der Erfindung

Aminosäuren, Vitamine und Nukleotide finden in der Tierernährung, in der Lebensmittelindustrie, in der pharmazeutischen Industrie und in der Humanmedizin Anwendung. Diese Stoffe werden mit Stämmen coryneformer Bakterien produziert. Zur Verbesserung der Produktionseigenschaften werden geeignete Gene mittels Plasmidvektoren verstärkt. Es besteht daher ein allgemeines Interesse, neue Plasmidvektoren mit verbesserten Eigenschaften zur Verfügung zu stellen.

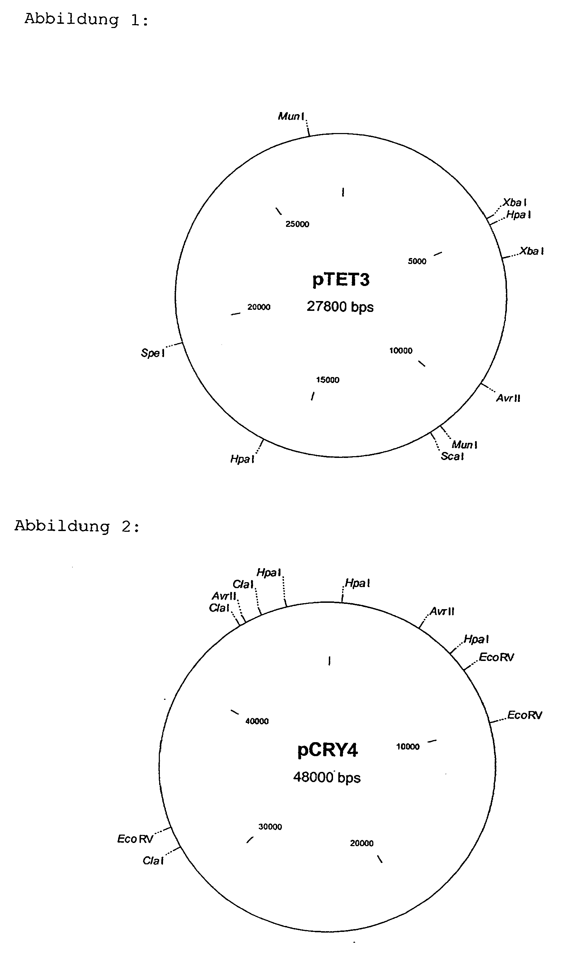

Gegenstand der Erfindung sind die miteinander kompatiblen Plasmide pTET3 und pCRY4, isoliert aus dem unter der DSM-Nummer 5816 hinterlegten Stamm von Corynebacterium glutamicum, wobei
1.1 das Plasmid pTET3 charakterisiert ist durch eine Länge von ~ 27,8 kbp und die in Abbildung 1 dargestellte Restriktionskarte, und eine Antibiotikaresistenzregion und
1.2 das Plasmid pCRY4 charakterisiert ist durch eine Länge von ~ 48 kbp, und die in Abbildung 2 dargestellte Restriktionskarte.

Gegenstand der Erfindung sind ebenso zur autonomen Replikation in coryneformen Bakterien befähigte zusammengesetzte Plasmide (composite plasmids), enthaltend
2.1 zumindest eine DNA-Replikationsregion, die sich von einem der Plasmide pTET3 oder pCRY4 ableitet (derived from)
2.2 gegebenenfalls ein Genfragment, das sich von einem Plasmid ableitet, das sich in E.coli, B.subtilis oder Streptomyces vermehren kann und
2.3 mindestens eine Region für die Expression einer Wirkstoffresistenz, bevorzugt aus dem Plasmid pTET3.

Gegenstand der Erfindung sind ebenfalls zusammengesetzte Plasmide, die aus den erfindungsgemäßen neuen Plasmiden zumindest die Wirkstoffresistenz(en) und pGA1 und/oder pGA2 ganz oder zum Teil enthalten.

Das neue Plasmid pTET3, dessen Restriktionskarte in Abbildung 1 dargestellt ist, besitzt
1. eine Replikationsregion mit der Nukleotidsequenz dargestellt in SEQ-ID-No. 1 und
2. eine Antibiotikaresistenzregion, bestehend aus einem Tetracyclinresistenz-vermittelndem tetA-Gen und einem Streptomycin- und Spectinomycinresistenzvermittelnden aadA-Gen, dargestellt in SEQ-ID-No. 6.

Das neue Plasmid pCRY4, dessen Restriktionskarte in Abbildung 2 dargestellt ist, besitzt eine Replikationsregion mit der Nukleotidsequenz, dargestellt in SEQ-ID-No. 4 enthält.

Die Herstellung von Aminosäuren, Vitaminen und Nukleotiden unter Verwendung von Plasmidvektoren (composite plasmids), die pTET3- und pCRY4- und gegebenenfalls pGA1- oder pGA2-Nukleotidsequenzen enthalten, ist ebenso ein Gegenstand der Erfindung.

Corynebacterium glutamicum LP-6, das im Rahmen der EP-B 0 472 869 als DSM5816 hinterlegt worden ist, enthält neben den dort beschriebenen Plasmiden pGA1 und pGA2 die neuen Plasmide pTET3 und pCRY4. Die Aufbewahrungszeit von DSM5816 ist gemäß Regel 9.1 des Budapester Vertrages verlängert worden.

Zur Darstellung der Plasmide pTET3 und pCRY4 wird der Stamm LP-6 in einem herkömmlichen Medium, wie z.B. Hirn-Herz-Bouillon oder Luria-Bertani-Medium, kultiviert. Die Zellen werden durch Zentrifugation geerntet, mit Lysozym behandelt und mit der Methode der alkalischen Lyse aufgeschlossen. Die DNA wird anschließend durch Anionenaustauschchromatographie an Silikagelpartikeln gereinigt, mit Ethanol oder Isopropanol gefällt und abschließend in H₂O resuspendiert. Vollständige Systeme zur Isolierung von Plasmid-DNA sind als sogenannte "Kits" im Handel erhältlich. Ein Beispiel für einen derartigen "Kit" ist der "Nucleobond Plasmid Kit" der Firma Clontech Laboratories GmbH. Eine detaillierte Anleitung zur Benutzung dieses "Kits" findet der Fachmann im Handbuch "NucleoBond Nucleic Acid Purification Kits and Cartridges, User Manual (PT3167-1)" der Firma Clonetech Laboratories GmbH (Heidelberg, Deutschland, 1997). Durch Auftrennung der auf diese Weise gewonnenen Gesamtplasmid-DNA durch Agarosegel-Elektrophorese und Färbung mittels Ethidiumbromid werden die Plasmide pTET3 und pCRY4 als sogenannte Plasmidbanden sichtbar. DNA des Plasmids pTET3 und DNA des Plasmids pCRY4 kann anschließend aus dem Agarosegel isoliert werden. Hierzu wird das Plasmid-DNA enthaltende Agarosegel mit einem chaotropen Reagenz versetzt, die in der entstehenden Lösung enthaltende Plasmid-DNA an die Oberfläche von Glas- oder Silikagelpartikeln gebunden und anschließend von dieser Matrix wieder eluiert. Eine detaillierte Anleitung hierzu findet findet der Fachmann im Handbuch "QIAEX II Handbook for DNA Extraction from Agarose Gels" der Firma Qiagen GmbH (Hilden, Deutschland, 1997). Auf die beschriebene Weise gelingt es, pTET3- und pCRY4-DNA in reiner Form darzustellen.

DNA des zu untersuchenden Plasmids wird mit Restriktionsenzymen wie sie beispielsweise bei Roberts et al. (Nucleic Acids Research 27, 312-313 (1999)) beschrieben sind einzeln oder in Kombinationen behandelt. Die entstehenden DNA-Fragmente werden durch Agarosegel-Elektrophorese aufgetrennt und die Restriktionsschnittstellen einander zugeordnet. Anleitungen hierzu findet der Fachmann beispielsweise bei Rodriguez und Tait "Recombinant DNA Techniques: An Introduction" (Addison-Wesley Publishing Company, London, 1983) oder im "Guide to Molecular Cloning Techniques" herausgegeben von Berger und Kimmel (Methods in Enzymology, Vol. 152, Academic Press, London, 1987). Auf diese Weise läßt sich die Länge des Plasmids bestimmen bzw. die Restriktionskarte aufstellen. Plasmid pTET3 hat eine Länge von ca. 27,8 kbp und ist in Abbildung 1 dargestellt. Plasmid pCRY4 hat eine Länge von ca. 48 kbp und ist in Abbildung 2 dargestellt.

Die Plasmide pTET3 und pCRY4 besitzen eine moderate bzw. niedrige Kopienzahl. Durch diese Eigenschaft ergänzen sie das Spektrum der bekannten Plasmide von Corynebacterium in vorteilhafter Weise. Anleitungen zur Kopienzahlbestimmung finden sich beispielsweise bei Miwa et al. (Agricultural and Biological Chemistry 48, 2901-2903 (1984)) und Vohradsky et al. (Electrophoresis 13, 601-612 (1993)).

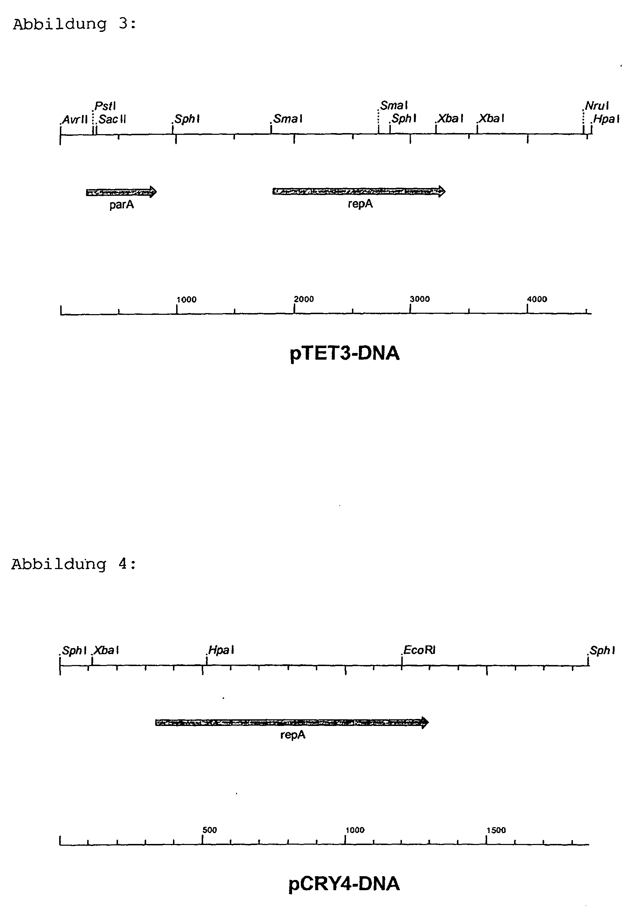

Zur Gewährleistung einer einfachen Handhabbarkeit der Plasmide pTET3 und pCRY4 wird die für die Replikation zuständige DNA-Region der jeweiligen Plasmide bestimmt. Hierzu bedient man sich bekannter Plasmidvektoren von Escherichia coli wie beispielsweise pK18 (Pridmore, Gene 56, 309-312 (1987)), pK18mob2 (Tauch et al., Plasmid 40, 126-139 (1998)) oder pCR2.1 (Invitrogen BV, Groningen, Niederlande), die in coryneformen Bakterien nicht replizieren können, deren Resistenzgen aber exprimiert wird. DNA der Plasmide pTET3 und pCRY4 wird isoliert und mit Restriktionsenzymen behandelt. Wahlweise können wiederum einzelne der auf diese Weise entstandenen DNA-Fragmente isoliert werden. Die DNA der eingesetzten Plasmidvektoren wird mit den gleichen Restriktionsenzymen bzw. mit solchen behandelt, die kompatible Enden erzeugen. Die erzeugten DNA-Moleküle werden gemischt und mit T4-DNA-Ligase behandelt. Diese sogenannten Kloniertechniken gehören zum Stand der Technik und sind beispielsweise in dem bekannten Handbuch von Sambrook et al. (Molecular Cloning: A Laboratory Manual, Cold Spring Harbor Laboratory Press (1989)) detailliert beschrieben. Nach Transformation eines coryneformen Wirtes, z.B. Corynebacterium glutamicum, mit dem Ligationsgemisch und Selektion auf das Resistenzgen des eingesetzten E. coli-Plasmidvektors erhält man Transformanten. Anleitungen zur Transformation coryneformer Bakterien findet man beispielsweise bei Thierbach et al. (Applied and Environmental Microbiology 29, 356-362 (1988)), bei Liebl et al. (FEMS Microbiology Letters 65, 299-304 (1989)) oder bei Dunican et al. (Bio/Technology 7, 1067-1070 (1989)). Die Plasmid-DNA dieser Transformanten enthält DNA-Segmente von pTET3 bzw. pCRY4, die zur Replikation in coryneformen Bakterien befähigen. Beispiele hierfür sind:
- das Plasmid pTET3-Rep, das aus dem E. coli-Plasmid pK18mob2 und der Replikationsregion des Plasmids pTET3 (Abbildung 3) besteht, und
- das Plasmid pCRY4-Rep, das aus dem E. coli-Plasmid pK18mob2 und der Replikationsregion des Plasmids pCRY4 (Abbildung 4) besteht.

Die auf diese Weise charakterisierten DNA-Abschnitte werden anschließend wiederum in gängige für die DNA-Sequenzierung geeignete Vektoren subkloniert. Beispiele für derartige, für die DNA-Sequenzierung geeignete Vektoren sind beispielsweise die Plasmide pGEM-5zf(-) oder pGEM-5zf(+) der Firma Promega Corporation (Promega Protocols and Application Guide, Second Edition, 1991, part number Y981, Promega Corporation, Madison, WI, USA), das Plasmid pUC19 (Yanish-Perron et al., Gene 33, 103-119 (1985)) oder das Plasmid pK18 (Pridmore, Gene 56, 309-312 (1987)).

Methoden zur DNA-Sequenzierung sind unter anderem bei Sanger et al. (Proceedings of the National of Sciences USA, 74, 5463-5467, 1977)) und bei Zimmermann et al. (Nucleic Acids Research 18, 1067 (1990)) beschrieben.

Die erhaltenen DNA-Sequenzen können dann mit bekannten Algorithmen bzw. Sequenzanalyse-Programmen, wie z.B. dem "STADEN Computer-Softwarepaket" (Molecular Biotechnology 5, 233-241 (1996)), dem GCG-Programm von Butler (Methods of Biochemical Analysis 39, 74-97 (1998)), dem FASTA-Algorithmus von Pearson und Lipman (Proceedings of the National Academy of Sciences USA 85, 2444-2448 (1988)) oder dem BLAST-Algorithmus von Altschul et al. (Nature Genetics 6, 119-129 (1994)) untersucht und mit den in öffentlich zugänglichen Datenbanken vorhandenen Sequenzeinträgen verglichen werden. Öffentlich zugängliche Datenbanken für Nukleotidsequenzen sind beispielsweise die der European Molecular Biologies Laboratories (EMBL, Heidelberg, Deutschland) oder die des National Center for Biotechnology Information (NCBI, Bethesda, MD, USA).

Auf diese Weise wurde die neue für die Replikation des Plasmids pTET3 verantwortliche DNA-Sequenz erhalten, die als SEQ ID No. 1 Bestandteil der vorliegenden Erfindung ist, und die das für die Replikation zuständige repA-Gen und das für die Stabilität zuständige parA-Gen trägt. Weiterhin wurden aus der vorliegenden DNA-Sequenz die Aminosäuresequenzen der kodierten Proteine abgeleitet. In SEQ ID No. 2 ist die sich ergebende Aminosäuresequenz des Stabilisierungsproteins ParA und in SEQ ID No. 3 die sich ergebende Aminosäuresequenz des Replikationsproteins RepA von pTET3 dargestellt.

Weiterhin wurde auf diese Weise die neue für die Replikation des Plasmids pCRY4 verantwortliche DNA-Sequenz erhalten, die als SEQ ID No. 4 Bestandteil der vorliegenden Erfindung ist, und die das für die Replikation von pCRY4 zuständige repA-Gen trägt. In SEQ ID No. 5 ist die abgeleitete Aminosäuresequenz des Replikationsproteins RepA des Plasmids pCRY4 dargestellt.

In Corynebacterium glutamicum sind natürlich vorkommende Gene, die Resistenz gegen Antibiotika vermitteln, nur als Ausnahme bekannt. Um so überraschender war es für die Erfinder festzustellen, daß das Plasmid pTET3 Resistenz gegen die Antibiotika Tetracyclin, Streptomycin, Spectinomycin und Sulfamethoxazol vermittelt.

Zur Identifizierung von Antibiotikum-Resistenzgenen auf neuen Plasmiden werden der zu untersuchende Stamm, im vorliegenden Fall Corynebacterium glutamicum LP-6, und ein sensitiver Kontrollstamm, im vorliegenden Fall Corynebacterium glutamicum ATCC13032, zunächst auf Resistenz bzw. Sensitivität gegen verschiedene Antibiotika und Antibiotika-Konzentrationen geprüft. Hierzu bedient man sich vorzugsweise der experimentellen Vorschriften des "National Committee of Clinical Laboratory Standards" (NCCLS) (Methods for dilution antimicrobial susceptibility tests for bacteria that grow aerobically-fourth edition; Approved Standard, M7-A4, NCCLS 17(2), (1997)). Den Vorschriften der "Approved Standards M7-A4" folgend lassen sich Hemmkonzentrationen ermitteln und somit Resistenzen des untersuchten Bakterienstammes identifizieren.

Anschließend wird das zu untersuchende Plasmid, im vorliegenden Fall pTET3, wie oben beschrieben aus Stamm LP-6 isoliert und zur Transformation eines geeigneten Kontroll- bzw. Indikatorstammes, im vorliegenden Fall Stamm ATCC13032, eingesetzt. Methoden zur Transformation coryneformer Bakterien sind beispielsweise bei Thierbach et al. (Applied and Environmental Microbiology 29, 356-362 (1988)), bei Liebl et al. (FEMS Microbiology Letters 65, 299-304 (1989)) oder bei Dunican et al. (Bio/Technology 7, 1067-1070 (1989)) beschrieben. Die Selektion erfolgt auf gängigen, komplexen Nährböden, wie z.B. Hirn-Herz-Bouillon oder Luria-Bertani-Medium, die mit den entsprechenden Antibiotika supplementiert werden. Das Antibiotikum und dessen Konzentration für diesen Selektionsvorgang wird mit Hilfe der oben erwähnten "Approved Standards, M7-A4" bestimmt. Auf diese Weise erhält man durch Selektion auf Tetracyclinresistenz den Stamm ATCC13032 [pTET3]. Anschließend wird die Resistenz/Sensitivität des Stammes ATCC13032 [pTET3] und des Kontrollstammes ATCC13032 mit der oben zitierten Vorschrift untersucht, mit dem Ergebnis, daß der Stamm ATCC13032 [pTET3] resistent gegenüber den Antibiotika Tetracyclin, Streptomycin, Spectinomycin und Sulfamethoxazol ist.

Zur weiteren Charakterisierung der Antibiotikaresistenzen werden diese kloniert und sequenziert. Hierzu wird das Plasmid pTET3 aus Stamm LP-6 oder ATCC13032 [pTET3] isoliert, mit geeigneten Restriktionsenzymen behandelt, mit in gleicher Weise behandelten Kloniervektoren gemischt und mit T4-DNA-Ligase behandelt. Das Ligationsgemisch wird durch Transformation in einen geeigneten Klonierwirt von Escherichia coli überführt. Die Selektion auf Transformanten erfolgt auf einem komplexen Nährboden, der mit dem jeweiligen Antibiotikum supplementiert wird. Methodische Anleitungen hierzu findet der Fachmann beispielsweise in dem bekannten Handbuch von Sambrook et al.. Geeignete Kloniervektoren sind beispielsweise pUC19 (Yanish-Perron et al., Gene 33, 103-119 (1985)), pK18mob2 (Tauch et al., Plasmid 40, 126-139 (1998)) oder pCR2.1 (Invitrogen BV, Groningen, Niederlande). Als Wirte eignen sich besonders solche E. coli-Stämme, die restriktions- und rekombinationsdefekt sind. Ein Beispiel hierfür ist der Stamm DH5αMCR, der von Grant et al. (Proceedings of the National Academy of Sciences USA 87, 4645-4649 (1990)) beschrieben wurde. Transformationsmethoden sind beispielsweise bei Hanahan (Journal of Molecular Biology 166, 577-580 (1983)) oder Tauch et al. (Plasmid 40, 126-139 (1998)) beschrieben. Zur Selektion auf Transformanten werden die Antibiotika verwendet, gegen die das Plasmid pTET3 Resistenz verleiht. Die Plasmid-DNA der erhaltenen Transformanten wird anschließend isoliert und die klonierten DNA-Fragmente des Plasmids pTET3 werden sequenziert. Die Sequenzen werden anschließend wie oben beschrieben analysiert und mit in Datenbanken gesammelten DNA-Sequenzen verglichen.

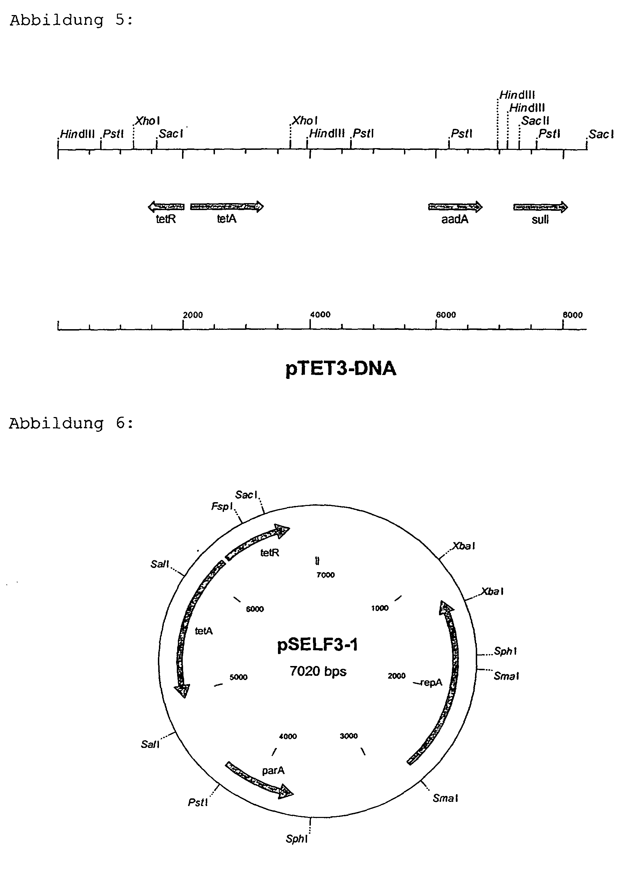

Die Erfinder fanden auf diese Weise heraus, daß die Gene die Resistenz gegen die Antibiotika Tetracyclin, Streptomycin, Spectinomycin und Sulfamethoxazol vermitteln, auf einem kontinuierlichen DNA-Fragment lokalisiert sind. Dieses DNA-Fragment ist als Restriktionskarte in Abbildung 5 dargestellt. Der DNA-Abschnitt, der die Gene tetR, tetA und aadA enthält, ist als Sequenz in der SEQ ID No. 6 dargestellt und Bestandteil der Erfindung.

Weiterhin wurden die Aminosäuresequenzen des von dem jeweiligen Gen kodierten Proteins aus der ermittelten DNA-Sequenz abgeleitet. In SEQ ID No. 7 ist die abgeleitete Aminosäuresequenz des vom tetA-Gen kodierten Tetracyclin-Resistenzproteins TetA und in SEQ ID No. 8 die abgeleitete Aminosäuresequenz des vom aadA-Gen kodierten Spectinomycin/ Streptomycin-Resistenzproteins AadA dargestellt. SEQ ID No. 9 zeigt die Kodierregion des tetR-Gens und SEQ ID No. 10 die Aminosäuresequenz des Tetracyclinresistenz-Repressorproteins TetR.

Kodierende DNA-Sequenzen, die sich aus SEQ ID No. 6 durch die Degeneriertheit des genetischen Codes ergeben, sind ebenfalls Bestandteil der Erfindung. In gleicher Weise sind DNA-Sequenzen, die mit SEQ ID No. 1 oder Teilen von SEQ ID No. 1 hybridisieren, Bestandteil der Erfindung. In der Fachwelt sind weiterhin konservative Aminosäureaustausche wie z.B. der Austausch von Glycin gegen Alanin oder von Asparaginsäure gegen Glutaminsäure in Proteinen als "Sinnmutationen" (sense mutations) bekannt, die zu keiner grundsätzlichen Veränderung der Aktivität des Proteins führen, d. h. funktionsneutral sind. Aminosäuresequenzen, die sich in entsprechender Weise aus SEQ ID No. 7, 8 und 10 ergeben sind ebenfalls Bestandteil der Erfindung.

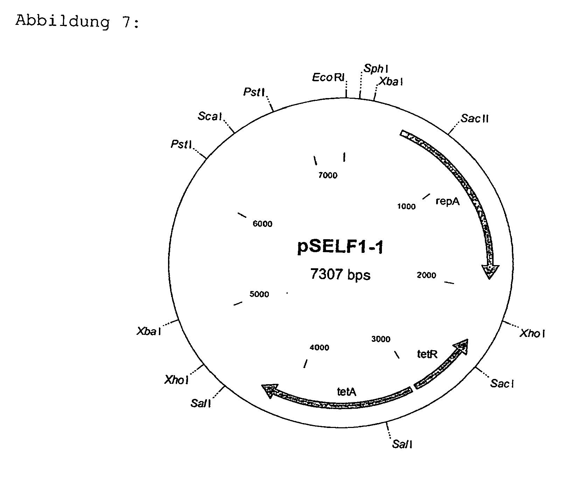

Die DNA-Fragmente der Plasmide pTET3 und pCRY4 aus Corynebacterium glutamicum Stamm LP-6 können anschließend mit DNA-Fragmenten bekannter Plasmide anderer Mikroorganismen, wie z.B. Escherichia coli oder Corynebacterium glutamicum, zu weiteren, neuen Plasmidvektoren kombiniert werden. Im Rahmen der vorliegenden Erfindung wird die Verwendung von Plasmid-DNA anderer Stämme der Spezies Corynebacterium glutamicum bevorzugt. Diese als Selbstklonierung bezeichnete Vorgehensweise hat den Vorteil, daß das Einbringen artfremder Nukleotidsequenzen in die Spezies Corynebacterium glutamicum unterbleibt. Derartige, weiterentwickelte Plasmidvektoren können ausschließlich aus Bestandteilen des neuen Plasmids pTET3 bestehen, d.h. aus einer Replikationsregion und mindestens einer Antibiotikum-Resistenzregion, die als Selektionsmarker benutzt wird. Ein Beispiel hierfür ist der in Abbildung 6 dargestellte Plasmidvektor pSELF3-1. Sie können aber auch aus Bestandteilen eines bekannten Plasmids und Bestandteilen von pTET3 oder pCRY4 zusammengesetzt werden. Ein Beispiel hierfür ist der in Abbildung 7 dargestellte Plasmidvektor pSELF1-1, bei dem das bekannte kryptische Plasmid pGA1 (US-A 5,175,108) mit dem Tetracyclinresistenz-vermittelnden tetA-Gen von pTET3 versehen wurde.

Die ausgehend von den neuen Plasmiden pTET3 und pCRY4 konstruierten Plasmidvektoren lassen sich vorteilhaft für die fermentative Herstellung industriell interessanter Stoffwechselprodukte wie Aminosäuren, Vitamine und Nukleotide einsetzen.

Beispielhaft wurde im Rahmen der vorliegenden Erfindung ein für eine "feed back"-resistente Aspartatkinase kodierendes lysC(FBR)-Allel von C. glutamicum mittels pSELF1-1 in C. glutamicum ATCC13032 kloniert. Auf diese Weise wurde ein selbstklonierter Lysin-produzierender Stamm von C. glutamicum hergestellt.

Weiterhin wurde beispielhaft das für die L-Aspartat-α-Decarboxylase kodierende panD-Gen von C. glutamicum mittels pSELF1-1 in den C. glutamicum Stamm ATCC13032ΔilvA kloniert. Auf diese Weise wurde ein selbstklonierter Pantothensäure-produzierender Stamm von C. glutamicum hergestellt.

Ein ganz besonderer Vorteil der neuen Plasmide pTET3 und pCRY4 und davon ausgehend hergestellter weiterer Plasmidvektoren besteht darin, daß sie einen ungewöhnlich hohen Grad an Kompatatibilität zu bekannten Plasmiden bzw. Plasmidvektoren aufweisen.

So wurde herausgefunden, daß Plasmid pTET3 in Gegenwart von Plasmidvektoren, die auf pGA1 (US-A 5,175,108), pAG3 (US-A 5,158,891), pBL1 (Santamaria et al., Journal of General Microbiology 130, 2237-2246 (1984)) oder auf pHM1519 (Miwa et al., Agricultural and Biological Chemistry 48, 2901-2903 (1984)) beruhen, koexistieren kann bzw. mit diesen kompatibel ist. Diese Kompatibilität von pTET3 ist auch dann gegeben, wenn die betreffende Wirtszelle bereits mehrere der bekannten Plasmidvektoren beispielsweise einen pBL1-Abkömmling und gleichzeitig einen pHM1519-Abkömmling enthält. Die Fähigkeit von pTET3 mit bekannten Plasmiden bzw. Plasmidvektoren koexistieren zu können, ist über einen ausreichend langen Zeitraum bzw. für eine ausreichend große Anzahl an Generationen gewährleistet.

Weiterhin wurde herausgefunden, daß Plasmid pCRY4 bei gleichzeitiger Anwesenheit der Plasmide pTET3, pGA1 (US-A 5,175,108) und pGA2 (US-A 5,175,108) in Gegenwart von Plasmidvektoren, die auf pAG3 (US-A 5,158,891), pBL1 (Santamaria et al., Journal of General Microbiology 130, 2237-2246 (1984)) oder auf pHM1519 (Miwa et al., Agricultural and Biological Chemistry 48, 2901-2903 (1984)) beruhen, koexistieren kann bzw. mit diesen kompatibel ist. Diese Kompatibilität von pCRY4 ist auch dann gegeben, wenn die betreffende Wirtszelle bereits mehrere der bekannten Plasmidvektoren beispielsweise einen pBL1-Abkömmling und gleichzeitig einen pHM1519-Abkömmling enthält. Die Fähigkeit von pCRY4 mit bekannten Plasmiden bzw. Plasmidvektoren koexistieren zu können, ist über einen ausreichend langen Zeitraum bzw. für eine ausreichend große Anzahl an Generationen gewährleistet.

Die hohe Kompatibilität der Plasmide pTET3 und pCRY4 kann in vorteilhafter Weise zur Verbesserung von Stämmen eingesetzt werden, die Aminosäuren, Vitamine und Nukleotide produzieren. So beschreiben Sahm und Eggeling (Applied and Environmental Microbiology 65, 1973-1979 (1999)) beispielsweise den Pantothensäure-produzierenden Stamm ATCC13032ΔilvA [pECM3ilvBNCD, pEKEx2panBC]. Dieser Stamm trägt das pHM1519-Derivat pECM3ilvBNCD und das pBL1-Derivat pEKEx2panBC. Der genannte Stamm, der bereits zwei Plasmide enthält, konnte nach Transfer des panD-Gens mittels des Plasmidvektors pSELF3-1 in seinen Leistungseigenschaften deutlich verbessert werden.

### Beispiele

Die vorliegende Erfindung wird im folgenden anhand von Ausführungsbeispielen näher erläutert.

### Folgende Bakterienstämme wurden verwendet:

Corynebacterium glutamicum LP-6 wurde im Rahmen von EP-B 0 472 869 bei der Deutschen Sammlung von Mikroorganismen und Zellkulturen (DSMZ, Braunschweig, Deutschland) als DSM5816 hinterlegt. Die Aufbewahrungszeit von DSM5816 wurde gemäß Regel 9.1 des Budapester Vertrages verlängert. DSM5816 hat die folgenden taxonomischen Merkmale:
- Zellform: Y-förmige Verzweigung
- Peptidoglycan: meso-Diaminopimelinsäure
- Mycolsäuren: Corynebacterium-Mycolsäuren mit hoher Ähnlichlichkeit zu DSM20300
- Fettsäuremuster: für Corynebacterium typisches Fettsäuremuster mit unverzweigten, gesättigten und ungesättigten Fettsäuren mit hoher Ähnlichkeit zu dem von DSM20300.
- G+C Gehalt: 55,1%
- 16S rDNA Sequenz: 98,6% Identität im Vergleich zu DSM20300
- DNA-DNA Homologie: 81,6% zu DSM20300

Corynebacterium glutamicum ATCC13032 wurde von der American Type Culture Collection (Manassas, USA) bezogen.

Corynebacterium glutamicum ATCC13032ΔilvA ist als DSM12455 bei der Deutschen Sammlung von Mikroorganismen und Zellkulturen (DSMZ, Braunschweig, Deutschland) hinterlegt.

Die in den nachstehenden Beispielen genannten allgemeinen genetischen Arbeitstechniken und verwendeten Nährmedien sind in der Fachliteratur bei Sambrook et al. (Molecular Cloning: A Laboaratory Manual, Cold Spring Harbor Laboratory Press (1989)) beschrieben. Der Elektrotransfer von Plasmid-DNA erfolgte nach der Methode von Liebl et al. (FEMS Microbiology Letters 65, 299-304 (1989)).

Die Sequenzierung der in den nachstehenden Beispielen beschriebenen DNA-Fragmente erfolgte nach der Dideoxy-Kettenabbruchmethode von Sanger et al. (Proceedings of the National Academy of Sciences USA 74, 5463-5467 (1977)). Die erhaltenen Sequenz-Rohdaten wurden unter Benutzung des "STADEN Software-Paketes" (Staden, Molecular Biotechnology 5, 233-241 (1996)) prozessiert. Die computergestützten Kodierbereichsanalysen wurde mit dem Programm "XNIP" (Staden, Molecular Biotechnology 5, 233-241 (1996)) durchgeführt. Weitere Sequenzanalysen wurden mit den "BLAST-Programmen" (Altschul et al., Nucleic Acids Research 25, 3389-3402 (1997)) durchgeführt.

### Beispiel 1

### Isolierung und Charakterisierung der neuen Plasmide pTET3 und pCRY4

Zur Identifizierung neuer Plasmide und zur Isolierung von Plasmid-DNA wurde der Bakterienstamm Corynebacterium glutamicum LP-6 in LB-Medium angezogen und nach der Anleitung des "NucleoBond Nucleic Acid Purification Kits and Cartridges User Manual (PT3167-1)" (Clonetech Laboratories GmbH, Heidelberg, Deutschland, 1997) isoliert. Die isolierte Plasmid-DNA wurde in einem 0,8%igen Agarosegel aufgetrennt und die Plasmidbanden, die den neuen Plasmiden pTET3 und pCRY4 entsprachen, jeweils getrennt aus dem Agaraosegel reisoliert. Die experimentelle Arbeitsanleitung entsprach dem "QIAEX II Handbook for DNA Extraction from Agarose Gels)" (Qiagen GmbH, Hilden,

Deutschland, 1997). Anschließend wurde die reisolierte Plasmid-DNA von pTET3 mit den Restriktionsenzymen AvrII, MunI (New England Biolabs GmbH (Schwalbach, Deutschland), HpaI, ScaI, XbaI (Pharmacia Biotech Europe GmbH, Freiburg, Deutschland) und SpeI (Roche Diagnostics GmbH, Mannheim, Deutschland) entsprechend den Herstellerangaben jeweils einzeln und in Kombination verdaut. Die Restriktionsansätze wurden anschließend in einem 0,8%igen Agarosegel aufgetrennt. Durch einen Vergleich der erhaltenen DNA-Fragmente mit DNA-Fragmenten bekannter Länge (DNA Molecular Weight Marker X, Roche Diagnostics GmbH, Mannheim, Deutschland) wurde die in Abbildung 1 dargestellte Restriktionskarte des Plasmids pTET3 aus Corynebacterium glutamicum LP-6 bestimmt.

Die reisolierte DNA des neuen Plasmids pCRY4 aus Corynebacterium glutamicum LP-6 wurde mit den Restriktionsenzymen AvrII (New England Biolabs GmbH (Schwalbach, Deutschland), EcoRV, HpaI und ClaI (Pharmacia Biotech Europe GmbH, Freiburg, Deutschland) nach den Herstellerangaben jeweils einzeln und in Kombination verdaut. Die Restriktionsansätze wurden anschließend in einem 0,8%igen Agarosegel aufgetrennt. Durch einen Vergleich der erhaltenen DNA-Fragmente mit DNA-Fragmenten bekannter Länge (DNA Molecular Weight Marker X, Roche Diagnostics GmbH, Mannheim, Deutschland) wurde die in Abbildung 2 dargestellte Restriktionskarte des Plasmids pCRY4 aus Corynebacterium glutamicum LP-6 bestimmt.

### Beispiel 2

### Isolierung und Sequenzierung der Replikationsregion von Plasmid pTET3

Zur Isolierung einer DNA-Region, die zur stabilen Replikation der neuen Plasmide in coryneformen Bakterien benötigt wird, wurde zunächst durch eine alkalische Behandlung der Bakterienzellen Plasmid-DNA aus Corynebacterium glutamicum LP-6 isoliert. Die experimentelle Vorgehensweise ist detailliert in der Anleitung des "NucleoBond Nucleic Acid Purification Kits and Cartridges User Manual (PT3167-1)" (Clonetech Laboratories GmbH, Heidelberg, Deutschland, 1997) beschrieben. Die erhaltene DNA-Präparation von Corynebacterium glutamicum LP-6 wurde anschließend in einem 0,8%igen Agarosegel aufgetrennt und auf das Vorhandensein von Plasmidbanden untersucht. Die identifizierten Plasmidbanden von Corynebacterium glutamicum LP-6 wurden den bekannten Plasmiden pGA1 und pGA2 (US-A- 5,175,108) sowie den neuen Plasmiden pTET3 und pCRY4 zugeordnet. Die Plasmidbande, die dem neuen Plasmid pTET3 entsprach, wurde aus dem Agaraosegel reisoliert (Siehe Beispiel 1). Die experimentelle Arbeitsanleitung hierzu findet sich im "QIAEX II Handbook for DNA Extraction from Agarose Gels" (Qiagen GmbH, Hilden, Deutschland, 1997). Anschließend wurde die reisolierte Plasmid-DNA mit den Restriktionsenzymen AvrII (New England Biolabs GmbH (Schwalbach, Deutschland) und HpaI (Pharmacia Biotech Europe GmbH, Freiburg, Deutschland) verdaut und in den mit den Restriktionsenzymen XbaI und SmaI (Pharmacia Biotech Europe GmbH, Freiburg, Deutschland) gespaltenen Vektor pK18mob2 (Tauch et al., Plasmid 40, 126-139 (1998)) kloniert. Die DNA-Restriktion und die DNA-Ligation mit dem Enzym T4 DNA-Ligase (Roche Diagnostics GmbH, Mannheim, Deutschland) wurden entsprechend den Vorschriften der Hersteller durchgeführt. Dieses Ligationsgemisch wurde anschließend durch Elektroporation in den Stamm Corynebacterium glutamicum ATCC13032 transferiert. Die Selektion wurde auf LB-Agar mit 25 µg/ml Kanamycin durchgeführt. Nach Inkubation für 48 Stunden bei 30°C wurden Kolonien isoliert, die Plasmide enthielten. Die Anwesenheit von Plasmiden in den transformierten Bakterienzellen wurde durch eine alkalische Lysemethode, die den Anleitungen im "QIAGEN Plasmid Mini Handbook for Plasmid Mini Kit" (Qiagen GmbH, Hilden, Deutschland, 1997) folgte, nachgewiesen. Das isolierte Plasmid wurde pTET3-Rep genannt. Restriktionsanalysen von pTET3-Rep sowie ein Vergleich der erhaltenen Fragmentlängen mit DNA-Fragmenten bekannter Länge (DNA Molecular Weight Marker X, Roche Diagnostics GmbH, Mannheim, Deutschland) ergaben, daß pTET3-Rep aus dem Kloniervektor pK18mob2 besteht, der ein ca. 4500 Basenpaare (bp) großes DNA-Fragment aus pTET3 enthält.

Für die doppelsträngige DNA-Sequenzierung des ca. 4500 bp großen DNA-Fragments aus pTET3-Rep wurde die DNA nach der Anleitung des "NucleoBond Nucleic Acid Purification Kits and Cartridges User Manual (PT3167-1)" (Clonetech Laboratories GmbH, Heidelberg, Deutschland, 1997) isoliert. Die Sequenzierung und die anschließende Kodierbereichsanalyse ergab die Identifizierung von zwei offenen Leserahmen (ORFs) auf dem sequenzierten DNA-Fragment. In Abbildung 3 ist eine Restriktionskarte des sequenzierten DNA-Fragments von pTET3-Rep, die auch die Lage der beiden identifizierten ORFs zeigt, dargestellt. Analysen mit Hilfe der BLAST-Programme ergaben, daß ORF1 für ein als ParA bezeichnetes Stabilisierungsprotein kodiert, und daß ORF2 für ein als RepA bezeichnetes Replikationsprotein kodiert. ORF1 wurde entsprechend als parA-Gen und ORF2 als repA-Gen bezeichnet. Die DNA-Sequenz des klonierten Fragmentes ist in SEQ ID No. 1 wiedergegeben. Die aus der DNA-Sequenz abgeleitete Aminosäuresequenz des Stabilisierungsproteins ParA ist in SEQ ID No. 2 dargestellt und die abgeleitete Aminosäuresequenz des Replikationsproteins RepA in SEQ ID No. 3.

### Beispiel 3

### Bestimmung der Kopienzahl des pTET3-Replikons in Corynebacterium glutamicum ATCC13032

Zur Bestimmung der Kopienzahl des Plasmids pTET3-Rep wurde der Bakterienstamm Corynebacterium glutamicum ATCC13032 [pTET3-Rep] für 20 Stunden bei 30°C in 100 ml LB-Medium mit 25 µg/ml Kanamycin angezogen. Anschließend wurde aus 25 ml Bakterienkultur die Gesamt-DNA des Stammes nach der Methode von Tauch et al. (Plasmid 34, 119-131 (1995)) isoliert. Die gewonnene DNA wurde für 20 Minuten bei 37°C mit 20 µg/ml RNase/DNase-frei (Roche Diagnostics GmbH, Mannheim, Deutschland) behandelt und nach einer Phenolextraktion elektrophoretisch im 0.8%igen Agarosegel aufgetrennt. Das mit Ethidiumbromid gefärbte Agarosegel wurde unter UV-Licht mit einem Cybertech CS1 Kamerasystem (Cybertech GmbH, Berlin, Deutschland) photographiert und das Negativ-Bild mit einem HP Scanjet 6100 C/T Optical Scanner (Hewlett-Packard Co., Palo Alto, CA, USA) digitalisiert. Die Bandenintensität der DNA wurde mit dem Computersystem WinCam der Firma Cybertech GmbH (Berlin, Deutschland) densitometrisch quantifiziert. Die Berechnung der Kopienzahl erfolgte nach der Methode von Miwa et al. (Agricultural and Biological Chemistry 48, 2901-2903 (1984)) unter der Annahme einer Chromosomengröße von 3082 kb (Bathe et al., Molecular and General Genetics 252, 255-265 (1996)) und ergab für das Plasmid pTET3-Rep in Corynebacterium glutamicum ATCC13032 einen Wert von 15 Plasmiden pro Chromosom.

### Beispiel 4

### Isolierung und Sequenzierung der Replikationsregion von Plasmid pCRY4

Zur Isolierung des DNA-Bereichs, der zur stabilen Replikation des neuen Plasmids pCRY4 in coryneformen Bakterien benötigt wird, wurde durch eine alkalische Behandlung der Bakterienzellen Plasmid-DNA aus Corynebacterium glutamicum LP-6 isoliert. Die experimentelle Vorgehensweise ist der Anleitung des "NucleoBond Nucleic Acid Purification Kits and Cartridges User Manual (PT3167-1)" (Clonetech Laboratories GmbH, Heidelberg, Deutschland, 1997) zu entnehmen. Die erhaltene DNA-Präparation von Corynebacterium glutamicum LP-6 wurde anschließend in einem 0,8%igen Agarosegel aufgetrennt und auf das Vorhandensein einer pCRY4-Plasmidbande untersucht. Die identifizierte Plasmidbande, die dem neuen Plasmid pCRY4 entsprach, wurde danach aus dem Agaraosegel reisoliert (Siehe Beispiel 1). Die experimentelle Arbeitsanleitung findet sich im "QIAEX II Handbook for DNA Extraction from Agarose Gels" (Qiagen GmbH, Hilden, Deutschland, 1997). Anschließend wurde die reisolierte Plasmid-DNA mit dem Restriktionsenzym SphI (Pharmacia Biotech Europe GmbH, Freiburg, Deutschland) verdaut und in den mit dem Restriktionsenzym SphI gespaltenen Vektor pK18mob2 (Tauch et al., Plasmid 40, 126-139 (1998)) kloniert. Die DNA-Restriktion und die DNA-Ligation mit dem Enzym T4 DNA-Ligase (Roche Diagnostics GmbH, Mannheim, Deutschland) wurden nach Herstellerangaben durchgeführt. Das Ligationsgemisch wurde anschließend durch Elektrotransfer in den coryneformen Bakterienstamm Corynebacterium glutamicum ATCC13032 transferiert. Die Selektion wurde auf LB-Agar mit 25 µg/ml Kanamycin durchgeführt. Nach Inkubation für 48 Stunden bei 30°C wurden plasmidhaltige Kolonien isoliert. Das Vorhandensein von Plasmiden in den transformierten Bakterienzellen wurde durch eine alkalische Lysemethode, die den Anleitungen im "QIAGEN Plasmid Mini Handbook for Plasmid Mini Kit" (Qiagen

GmbH, Hilden, Deutschland, 1997) folgte, nachgewiesen. Das isolierte Plasmid wurde pCRY4-Rep genannt. Restriktionsanalysen von pCRY4-Rep sowie ein Vergleich der erhaltenen Fragmentlängen mit DNA-Fragmenten bekannter Länge (DNA Molecular Weight Marker X, Roche Diagnostics GmbH, Mannheim, Deutschland) ergaben, daß pCRY4-Rep ein ca. 1900 bp großes DNA-Fragment enthält.

Für die doppelsträngige DNA-Sequenzierung des ca. 1900 bp großen DNA-Fragments aus pCRY4-Rep wurde die DNA nach der Anleitung des "NucleoBond Nucleic Acid Purification Kits and Cartridges User Manual (PT3167-1)" (Clonetech Laboratories GmbH, Heidelberg, Deutschland, 1997) isoliert. Die Sequenzierung der DNA und eine computergestützte Kodierbereichsanalyse ermöglichte die Identifizierung von einem offenen Leserahmen (ORF1) auf dem sequenzierten DNA-Fragment. In Abbildung 4 ist die Restriktionskarte des sequenzierten DNA-Fragments von pCRY4-Rep, die auch die Lage des identifizierten ORFs zeigt, dargestellt. Analysen mit Hilfe der BLAST-Programme ergaben, daß ORF1 für ein Replikationsprotein (RepA) kodiert, der als repA-Gen bezeichnet wurde. Die DNA-Sequenz des klonierten Fragments ist in SEQ ID No. 4 wiedergegeben, die abgeleitete Aminosäuresequenz des Replikationsproteins RepA ist in SEQ ID No. 5 dargestellt.

### Beispiel 5

### Bestimmung der Kopienzahl des pCRY4-Replikons in Corynebacterium glutamicum ATCC13032

Zur Bestimmung der Kopienzahl des Plasmids pCRY4-Rep wurde der Bakterienstamm Corynebacterium glutamicum ATCC13032 [pCRY4-Rep] für 20 Stunden bei 30°C in 100 ml LB-Medium mit 25 µg/ml Kanamycin angezogen. Anschließend wurde aus 25 ml Bakterienkultur die Gesamt-DNA des Stammes nach der Methode von Tauch et al. (Plasmid 34, 119-131 (1995)) isoliert. Die gewonnene DNA wurde für 20 Minuten bei 37°C mit 20 µg/ml RNase/DNase-frei (Roche Diagnostics GmbH, Mannheim, Deutschland) behandelt und nach einer Phenolextraktion elektrophoretisch im 0.8%igen Agarosegel aufgetrennt. Das mit Ethidiumbromid gefärbte Agarosegel wurde unter UV-Licht mit einem Cybertech CS1 Kamerasystem (Cybertech GmbH, Berlin, Deutschland) photographiert und das Negativ-Bild mit einem HP Scanjet 6100 C/T Optical Scanner (Hewlett-Packard Co., Palo Alto, CA, USA) digitalisiert. Die Bandenintensität der DNA wurde mit dem Computersystem WinCam der Firma Cybertech GmbH (Berlin, Deutschland) densitometrisch quantifiziert. Die Berechnung der Kopienzahl erfolgte nach der Methode von Miwa et al. (Agricultural and Biological Chemistry 48, 2901-2903 (1984)) unter der Annahme einer Chromosomengröße von 3082 kb (Bathe et al., Molecular and General Genetics 252, 255-265 (1996)) und ergab für das Plasmid pCRY4-Rep in Corynebacterium glutamicum ATCC13032 einen Wert von 3 Plasmiden pro Chromosom.

### Beispiel 6

### Isolierung und Sequenzierung der Antibiotikum-Resistenzregion des Plasmids pTET3

Zur Identifizierung von Antibiotikum-Resistenzbereichen auf den neuen Plasmiden pTET3 oder pCRY4 wurden der resistente Teststamm Corynebacterium glutamicum LP-6 und der sensitive Kontrollstamm Corynebacterium glutamicum ATCC13032 zunächst gemäß den experimentellen Vorschriften des "National Committee of Clinical Laboratory Standards" (National Committee of Clinical Laboratory Standards, Methods for dilution antimicrobial susceptibility tests for bacteria that grow aerobically; Approved Standard, M7-A4 (1997)) in Gegenwart und Abwesenheit verschiedener Antibiotika und Antibiotikum-Konzentrationen angezogen. Die für diesen Test benötigten Antibiotika, u.a. die Antibiotika Tetracyclin, Spectinomycin, Streptomycin und Sulfamethoxazol, wurden von der Firma Sigma-Aldrich Chemie GmbH (Deisenhofen, Deutschland) bezogen und in den in den "Approved Standards M7-A4" angegebenen Konzentrationen eingesetzt. Das für diesen Test benötigte Nährmedium "MÜLLER-HINTON-Bouillon" wurde von der Firma Merck KgaA (Darmstadt, Deutschland) bezogen und entsprechend den Herstellerangaben eingesetzt. Den Vorschriften der "Approved Standards M7-A4" folgend ließen sich Hemmkonzentrationen bestimmen (Tabelle 1) und Resistenzen des Bakterienstammes Corynebacterium glutamicum LP-6 gegen die Antibiotika Tetracyclin, Spectinomycin, Streptomycin und Sulfamethoxazol identifizieren. Anschließend wurde die mit Hilfe einer alkalischen Lysemethode ("NucleoBond Nucleic Acid Purification Kits and Cartridges User Manual (PT3167-1)",Clonetech Laboratories GmbH, Heidelberg, Deutschland, 1997) aus Corynebacterium glutamicum LP-6 isolierte Plasmid-DNA per Elektrotransfer nach Corynebacterium glutamicum ATCC13032 transferiert. Bei der Primärselektion auf LB-Agar mit 5 µg/ml Tetracyclin wurde direkt auf das Vorhandensein der identifizierten Tetracyclin-Resistenz selektioniert. Das Vorhandensein eines Plasmids im transformierten Bakterienstamm Corynebacterium glutamicum ATCC13032 wurde anschließend durch eine alkalische Lysemethode nachgewiesen ("NucleoBond Nucleic Acid Purification Kits and Cartridges User Manual (PT3167-1)", Clonetech Laboratories GmbH, Heidelberg, Deutschland, 1997). Restriktionsanalysen der isolierten Plasmid-DNA sowie ein Vergleich der erhaltenen Fragmentlängen mit DNA-Fragmenten bekannter Länge (DNA Molecular Weight Marker X, Roche Diagnostics GmbH, Mannheim, Deutschland) und mit DNA-Fragmenten des Plasmids pTET3 ergaben, daß es sich bei dem transformierten, Tetracyclinresistenz-vermittelnden Plasmid um das Plasmid pTET3 handelt. Der transformierte Stamm wurde Corynebacterium glutamicum ATCC13032 [pTET3] genannt.

Ein erneuter Resistenztest mit dem isolierten, resistenten Teststamm Corynebacterium glutamicum ATCC13032 [pTET3] und dem sensitiven Kontrollstamm Corynebacterium glutamicum ATCC13032 gemäß den experimentellen Vorschriften des "National Committee of Clinical Laboratory Standards" in Gegenwart verschiedener Konzentrationen der Antibiotika Tetracyclin, Spectinomycin, Streptomycin und Sulfamethoxazol zeigte, daß der Teststamm Corynebacterium glutamicum ATCC13032 [pTET3] Resistenzen gegen diese Antibiotika besitzt (Tabelle 1).

**Tabelle 1**

| Minimale Inhibierungskonzentration (µg Antibiotikum pro ml) verschiedenener Corynebacterium glutamicum-Stämme | | | |
|---|---|---|---|
| Antibiotikum | ATCC13032 | LP-6 | ATCC13032 |
| | | | [pTET3] |
| Tetracyclin | ≤ 0,75 | ≤ 12 | ≤ 12 |
| Spectinomycin | ≤ 50 | > 2000 | > 2000 |
| Streptomycin | ≤ 0,5 | ≤ 100 | ≤ 100 |
| Sulfamethoxazol | ≤ 150 | ≤ 300 | ≤ 300 |

| | | | |
|---|---|---|---|
| Die Symbole haben folgende Bedeutung: > : Die minimale Inhibierungskonzentratiuon ist größer als der angegebene Wert. ≤ : Die minimale Inhibierungskonzentration ist kleiner oder gleich dem angegebenen Wert. | | | |

Zur weiteren Charakterisierung der Antibiotika-Resistenzen von pTET3 wurde die Plasmid-DNA mit Hilfe einer alkalischen Lysemethode ("NucleoBond Nucleic Acid Purification Kits and Cartridges User Manual (PT3167-1)",Clonetech Laboratories GmbH, Heidelberg, Deutschland, 1997) aus Corynebacterium glutamicum ATCC13032 [pTET3] reisoliert. Die Plasmid-DNA wurde dann mit den Restriktionsenzymen HindIII bzw. SacI (Pharmacia Biotech Europe GmbH, Freiburg, Deutschland) gespalten und in die Escherichia coli-Kloniervektoren pK18mob2 (Tauch et al., Plasmid 40, 126-139 (1998)] bzw. pUC19 (Pharmacia Biotech Europe GmbH, Freiburg, Deutschland) ligiert. Die DNA-Restriktion und die DNA-Ligation mit dem Enzym T4 DNA-Ligase (Roche Diagnostics GmbH, Mannheim, Deutschland) wurden entsprechend den Vorschriften der Hersteller durchgeführt. Der Ligationsansatz wurde anschließend per Elektroporation in den Bakterienstamm Escherichia coli DH5αMCR transferiert (Tauch et al., FEMS Microbiology Letters 123, 343-348 (1994)]. Nach Selektion auf LB-Agar mit 5 µg/ml Tetracyclin bzw. 250 µg/ml Spectinomycin wurden transformierte Kolonien erhalten, deren Plasmidvektoren DNA-Abschnitte aus dem Plasmid pTET3 enthielten. Das Vorhandensein der Plasmidvektoren wurde durch eine alkalische Lysemethode nachgewiesen ("QIAprep Miniprep Handbook for Purification of Plasmid DNA", Qiagen GmbH, Hilden, Deutschland, 1997). Restriktionsanalysen der isolierten Plasmid-DNA sowie ein Vergleich der erhaltenen Fragmentlängen mit DNA-Fragmenten bekannter Länge ergaben, daß das pTET3-H9 genannte, isolierte Plasmid aus dem Plasmidvektor pK18mob2 und einem ca. 4000 bp großen DNA-Fragment aus pTET3 besteht, und daß das pXCS10 genannte, isolierte Plasmid aus dem Plasmidvektor pUC19 (Pharmacia Biotech Europe GmbH, Freiburg, Deutschland) und einem ca. 6750 bp großen DNA-Fragment aus pTET3 besteht. Der aus der Klonierung mit dem Restriktionsenzym HindIII hervorgegangene Plasmidvektor pTET3-H9 vermittelt in Eschericha coli DH5αMCR Resistenz gegen Tetracyclin (5 µg/ml), der aus der Klonierung mit dem Restriktionsenzym SacI hervorgegangene Plasmidvektor pXCS10 verleiht Resistenzen gegen die Antibiotika Spectinomycin (250 µg/ml), Streptomycin (250 µg/ml) und Sulfamethoxazole (300 µg/ml). Ein Vergleich der Restriktionsanalysen der klonierten DNA-Fragmente von pTET3 in den Plasmidvektoren pTET3-H9 und pXCS10 zeigte außerdem, daß beide DNA-Fragmente um ca. 2400 bp überlappen und somit zu einem kontinuierlichen DNA-Strang von ca. 8350 bp Länge zusammengesetzt werden können.

Für die doppelsträngige DNA-Sequenzierung eines kontinuierlichen, ca. 7300 bp großen und die Resistenzen gegen Tetracyclin, Spectinomycin und Streptomycin verleihenden DNA-Fragmentes von pTET3 wurde DNA der Plasmide pTET3-H9 und pXCS10 nach der Anleitung des "QIAprep Miniprep Handbook for Purification of Plasmid DNA" (Qiagen GmbH, Hilden, Deutschland, 1997) isoliert. Nach Sequenzierung und Analyse der Sequenz konnten vier offene Leserahmen (ORFs) auf dem sequenzierten DNA-Fragment bestimmt werden. In Abbildung 5 ist eine Restriktionskarte des sequenzierten DNA-Bereichs von pTET3 sowie die Lage der identifizierten offenen Leserahmen (ORFs) dargestellt. Die Analysen ergaben, daß ORF1 ein für ein Tetracyclinresistenz-Repressorprotein (TetR) kodierendes tetR-Gen, ORF2 ein für ein Tetracyclin-Resistenzprotein (TetA) kodierendes tetA-Gen, ORF3 ein für ein Spectinomycin-/Streptomycin-Resistenzprotein (AadA) kodierendes aadA-Gen und ORF4 ein für ein Sulfamethoxazol-Resistenzprotein (SulI) kodierendes sulI-Gen darstellt. Die DNA-Sequenz der Resistenzregion von pTET3 ist in SEQ ID No. 6 wiedergegeben. Die aus den Sequenzdaten abgeleitete Aminosäuresequenz des Tetracyclin-Resistenzproteins (TetA) ist in SEQ ID No. 7 dargestellt und die aus den Sequenzdaten abgeleitete Aminosäuresequenz des Spectinomycin-/Streptomycin-Resistenzproteins (AadA) in SEQ ID No. 8. Der Kodierbereich des für das Tetracyclinresistenz-Repressorprotein (TetR) kodierenden tetR-Gens ist außerdem in SEQ ID No. 9 dargestellt und die abgeleitete Aminosäuresequenz in SEQ ID No. 10.

### Beispiel 7

### Koexistenz des Plasmids pTET3 mit bekannten coryneformen Plasmiden in Corynebacterium glutamicum ATCC13032

Zur Analyse der Koexistenz des neuen Plasmids pTET3 aus Corynebacterium glutamicum LP-6 mit bekannten coryneformen Plasmiden wurde der in Beispiel 6 erzeugte Bakterienstammm Corynebacterium glutamicum ATCC13032 [pTET3] verwendet.

Von diesem Stamm wurden elektrokompetente Zellen erzeugt, in die anschließend Plasmidvektoren, die aus bekannten Plasmiden coryneformer Bakterien und Selektionsmarker-Anteilen bestehen, transferiert wurden. Für diesen DNA-Transfer wurden die Plasmidvektoren, pGA1-KE12, pAG3-Xba, pEBM2 [Tauch et al., Archives of Microbiology 169, 303-312 (1998)), pECM2 (Tauch et al., FEMS Microbiology Letters 123, 343-348 (1994)) und pECM3 ausgewählt. Beim Plasmid pGA1-KE12 handelt es sich um eine EcoRI-Fusion des kryptischen Plasmids pGA1 aus Corynebacterium glutamicum LP-6 mit dem Vektor pK18mob2 (Tauch et al., Plasmid 40, 126-139 (1998)). Das Plasmid pAG3-Xba ist eine XbaI-Fusion von pAG3 und pK18mob2. Das Plasmid pECM3 stellt eine BamHI-BglII-Deletion von pECM2 dar. Der erfolgte Transfer der Kanamycinresistenz-vermittelnden Plasmidvektoren pGA1-KE12 (pGA1-Derivat), pAG3-Xba (pAG3-Derivat), pEBM2 (pBL1-Derivat) und pECM2 (pHM1519-Derivat) wurde auf LB-Agar mit 25 µg/ml Kanamycin selektioniert. Weiterhin wurde in den resultierenden Bakterienstamm Corynebacterium glutamicum ATCC13032 [pTET3, pEBM2], der die Plasmide pTET3 und pEBM2 trägt, das Chloramphenicolresistenz-vermittelnde Plasmid pECM3, ein pHM1519-Derivat, übertragen. Nach DNA-Transfer wurde auf LB-Agar mit 7,5 µg/ml Chloramphenicol (Sigma-Aldrich Chemie GmbH, Deisenhofen, Deutschland) selektioniert. Zur Bestätigung des erfolgten Plasmidtransfers wurde von den hergestellten Stämmen bzw. Transformanten Plasmid-DNA isoliert ("NucleoBond Nucleic Acid Purification Kits and Cartridges User Manual (PT3167-1)",Clonetech Laboratories GmbH, Heidelberg, Deutschland, 1997) und im 0,8%igen Agarosegel nachgewiesen.

Auf diese Weise wurden folgende Stämme von Corynebacterium glutamicum hergestellt:
- ATCC13032 [pTET3, pGA1-KE12]
- ATCC13032 [pTET3, pAG3-Xba]
- ATCC13032 [pTET3, pEBM2]
- ATCC13032 [pTET3, pECM2]
- ATCC13032 [pTET3, pEBM2, pECM3].

Zum weiteren Nachweis der Koexistenz des neuen Plasmids pTET3 mit bekannten Plasmidvektoren wurden die hergestellten Stämme zunächst für 24 Stunden bei 30°C in LB-Medium angezogen, das mit den jeweiligen Antibiotika (5 µg/ml Tetracyclin, 25 µg/ml Kanamycin und 10 µg/ml Chloramphenicol) supplementiert worden war. Jeweils 1 ml der Kulturen wurde anschließend zweimal in antibiotikumfreiem LB-Medium gewaschen. Von den gewaschenen Bakteriensuspensionen wurden Verdünnungsreihen in LB-Medium angelegt und Suspensionen von 0,1 ml, die 10⁴ Zellen enthielten, auf jeweils 100 ml antibiotikumfreies und antibiotikumhaltiges LB-Medium übertragen. Diese Kulturen wurden erneut bei 30°C über ca. 25 Generationen angezogen und das Wachstum durch Messung der optischen Dichte bei einer Wellenlänge von 580 nm mit einem Spectrophotometer (Pharmacia LKB Novaspec II, Pharmacia, Freiburg, Deutschland) verfolgt. Die Kulturen wurden dabei mindestens bis zu einer optischen Dichte von 8 (optische Dichte von 1 entspricht 4×10⁸ Zellen pro ml) angezogen. Anschließend wurde aus den Kulturen die Plasmid-DNA isoliert und im 0,8%igen Agarosegel aufgetrennt. Die erhaltenen Plasmidbanden waren unter beiden Kulturbedingungen, d.h. in An- und Abwesenheit der Antibiotika, identisch und zeigten jeweils das Vorhandensen des Plasmids pTET3 und des transformierten Plasmidvektors, d. h. pGA1-KE12, pAG3-Xba, pEBM2, pECM2, und pEBM2 plus pECM3 an.

### Beispiel 8

### Koexistenz des Plasmids pCRY4 mit anderen coryneformen Plasmiden in Corynebacterium glutamicum LP-6

Zur Analyse der Koexistenz des Plasmids pCRY4 mit bekannten coryneformen Plasmiden wurde Corynebacterium glutamicum LP-6 eingesetzt, in dem pCRY4 bereits mit den Plasmiden pGA1, pGA2 und pTET3 koexistiert.

In diesen Bakterienstamm wurden weitere Plasmidvektoren, die aus bekannten, coryneformen Plasmiden und Selektionsmarker-Anteilen bestehen, transferiert. Für diesen DNA-Transfer wurden die Plasmidvektoren pAG3-Xba, pEBM2 (Tauch et al., Archives of Microbiology 169, 303-312 (1998)), pECM2 (Tauch et al., FEMS Microbiology Letters 123, 343-348 (1994)) und pECM3 eingesetzt. Das Plasmid pECM3 stellt eine BamHI-BglII-Deletion von pECM2 dar. Der Transfer der Kanamycinresistenz-vermittelnden Plasmidvektoren pAG3-Xba (pAG3-Derivat), pEBM2 (pBL1-Derivat), und pECM2 (pHM1519-Derivat) wurde auf LB-Agar mit 25 µg/ml Kanamycin selektioniert. Weiterhin wurde in den resultierenden Bakterienstamm Corynebacterium glutamicum LP-6 [pEBM2], der die Plasmide pGA1, pGA2, pTET3, pCRY4 und pEBM2 trägt, das Chloramphenicolresistenz-vermittelnde Plasmid pECM3, ein pHM1519-Derivat, übertragen. Nach DNA-Transfer wurde auf LB-Agar mit 7,5 µg/ml Chloramphenicol selektioniert. Zur Bestätigung des erfolgreichen Plasmidtransfers wurde von den hergestellten Stämmen bzw. Transformanten Plasmid-DNA isoliert ("NucleoBond Nucleic Acid Purification Kits and Cartridges User Manual (PT3167-1)",Clonetech Laboratories GmbH, Heidelberg, Deutschland, 1997) und die Plasmide durch Elektrophorese im 0,8%igen Agarosegel nachgewiesen.

Auf diese Weise wurden folgende Stämme von Corynebacterium glutamicum hergestellt:
- LP-6 [pAG3-Xba]
- LP-6 [pEBM2]
- LP-6 [pECM2]
- LP-6 [pEBM2, pECM3].
(Es sei daran erinnert, daß der Rezipientenstamm Corynebacterium glutamicum LP-6 bereits die Plasmide pGA1, pGA2, pTET3 und pCRY4 enthält.)

Zum weiteren Nachweis der Koexistenz des Plasmids pCRY4 mit bekannten Plasmidvektoren wurden die hergestellten Stämme zunächst für 24 Stunden bei 30°C in LB-Medium angezogen, das mit den jeweiligen Antibiotika (5 µg/ml Tetracyclin, 25 µg/ml Kanamycin und 10 µg/ml Chloramphenicol supplementiert worden war). Jeweils 1 ml der Bakterienkulturen wurde anschließend zweimal in antibiotikumfreiem LB-Medium gewaschen. Von den gewaschenen Bakteriensuspensionen wurden Verdünnungsreihen in LB-Medium angelegt und Suspensionen von 0,1 ml, die 10⁴ Zellen enthielten, auf jeweils 100 ml antibiotikumfreies und antibiotikumhaltiges LB-Medium übertragen. Diese Kulturen wurden erneut bei 30°C über ca. 25 Generationen angezogen und das Wachstum durch Messung der optischen Dichte bei einer Wellenlänge von 580 nm mit einem Spectrophotometer (Pharmacia LKB Novaspec II, Pharmacia, Freiburg, Deutschland) verfolgt. Die Kulturen wurden dabei mindestens bis zu einer optischen Dichte von 8 (optische Dichte von 1 entspricht 4×10⁸ Zellen pro ml) angezogen. Anschließend wurde aus den Kulturen die Plasmid-DNA isoliert und im 0,8%igen Agarosegel aufgetrennt. Die erhaltenen Plasmidbanden waren unter selektiven und nichtselektiven Kulturbedingungen, d. h. in An- und Abwesenheit der Antibiotika identisch und zeigten jeweils das Vorhandensein der Plasmide pGA1, pGA2, pTET3 und pCRY4 sowie des transformierten Plasmidvektors, d.h. pAG3-Xba, pEBM2, pECM2 und pEBM2 plus pECM3 an.

### Beispiel 9

### Konstruktion des Plasmidvektors pSELF3-1 aus pTET3

Zur Konstruktion eines Plasmidvektors, der aussschließlich aus Komponenten des neuen Plasmids pTET3 besteht, wurde zunächst durch eine alkalische Behandlung der Bakterienzellen ("NucleoBond Nucleic Acid Purification Kits and Cartridges User Manual (PT3167-1)", Clonetech Laboratories GmbH, Heidelberg, Deutschland, 1997) GesamtPlasmid-DNA aus Corynebacterium glutamicum LP-6 isoliert. Die erhaltene DNA-Präparation wurde anschließend in einem 0,8%igen Agarosegel aufgetrennt. Die Plasmidbande, die dem neuen Plasmid pTET3 entsprach, wurde aus dem Agaraosegel reisoliert ("QIAEX II Handbook for DNA Extraction from Agarose Gels", Qiagen GmbH, Hilden, Deutschland). Danach wurde die reisolierte Plasmid-DNA mit dem Restriktionsenzym XhoI (Pharmacia Biotech Europe GmbH, Freiburg, Deutschland) nach den Herstellerangaben verdaut. Der Restriktionsansatz wurde im 0,8%igen Agarosegel aufgetrennt und es wurde ein ca. 2500 bp großes DNA-Fragment, auf dem nach den DNA-Sequenzdaten (Beispiel 6) die Tetracyclin-Resistenzregion lokalisiert ist, reisoliert. Anschließend wurde die isolierte pTET3-DNA mit den Restriktionsenzymen AvrII (New England Biolabs GmbH(Schwalbach, Deutschland) und HpaI (Pharmacia Biotech Europe GmbH, Freiburg, Deutschland) gespalten. Der Spaltungansatz wurde ebenfalls in einem 0,8%igen Agarosegel aufgetrennt, und es wurde das ca. 4500 bp große DNA-Fragment, auf dem nach den DNA-Sequenzinformationen die Replikationsregion von pTET3 lokalisiert ist, reisoliert. Die überstehenden DNA-Enden von beiden reisolierten DNA-Fragmenten wurden danach mit dem Enzym Klenow-Polymerase aufgefüllt. Die Auffüllreaktion mit dem Enzym Klenow-Polymerase wurde nach den Herstellerangaben durchgeführt (Roche Diagnostics GmbH, Mannheim, Deutschland). Die aufgefüllten DNA-Fragmente wurden dann durch das Enzym T4 DNA-Ligase (Roche Diagnostics GmbH, Mannheim, Deutschland) nach Herstellerangaben miteinander ligiert. Das Ligationsgemisch wurde durch Elektroporation in Corynebacterium glutamicum ATCC13032 transferiert. Die Selektion wurde auf LB-Agar mit 5 µg/ml Tetracyclin durchgeführt. Nach Inkubation für 48 Stunden bei 30°C wurden Kolonien isoliert, die den neuen Plasmidvektor enthalten. Die Anwesenheit des Plasmidvektors in den transformierten Bakterienzellen wurde durch eine alkalische Lysemethode ("QIAGEN Plasmid Mini Handbook for Plasmid Mini Kit", Qiagen GmbH, Hilden, Deutschland, 1997) nachgewiesen. Das isolierte Plasmid wurde pSELF3-1 genannt. Restriktionsanalysen von pSELF3-1 sowie ein Vergleich der erhaltenen Fragmentlängen mit DNA-Fragmenten bekannter Länge ergaben die Restriktionskarte, die als Abbildung 6 beigefügt ist.

Plasmid pSELF3-1 besteht durch dieses Konstruktionsschema ausschließlich aus DNA-Fragmenten des neuen Plasmids pTET3 und damit aus DNA, die ausschließlich aus Corynebacterium glutamicum stammt.

### Beispiel 10

### Konstruktion des Plasmidvektors pSELF1-1

Aus dem bekannten Plasmid pGA1 (US-A 5,175,108) wurde unter Verwendung des Tetracyclinresistenzgens aus pTET3 (Siehe Beispiel 1 und 6) der Plasmidvektor pSELF1-1 hergestellt.

Hierzu wurde zunächst durch eine alkalische Behandlung der Bakterienzellen ("NucleoBond Nucleic Acid Purification Kits and Cartridges User Manual (PT3167-1)", Clonetech Laboratories GmbH, Heidelberg, Deutschland, 1997) GesamtPlasmid-DNA von Corynebacterium glutamicum LP-6 isoliert. Die erhaltene DNA-Präparation wurde in einem 0,8%igen Agarosegel aufgetrennt. Die Plasmidbanden, die dem bekannten Plasmid pGA1 und dem neuen Plasmid pTET3 entsprachen, wurden aus dem Agarosegel isoliert ("QIAEX II Handbook for DNA Extraction from Agarose Gels", Qiagen GmbH, Hilden, Deutschland). Danach wurde die isolierte Plasmid-DNA von pGA1 mit dem Restriktionsenzym SalI (Pharmacia Biotech Europe GmbH, Freiburg, Deutschland) nach den Herstellerangaben gespalten. Die isolierte Plasmid-DNA von pTET3 wurde mit dem Restriktionsenzym XhoI (Pharmacia Biotech Europe GmbH, Freiburg, Deutschland) gespalten. Der Restriktionsansatz von pTET3 wurde im 0,8%igen Agarosegel aufgetrennt und es wurde ein ca. 2500 bp großes DNA-Fragment, auf dem nach den DNA-Sequenzdaten (Beispiel 6) die Tetracyclin-Resistenzregion lokalisiert ist, reisoliert. Das erzeugte DNA-Fragment von pGA1 und das reisolierte DNA-Fragment von pTET3 wurden dann mittels T4 DNA-Ligase (Roche Diagnostics GmbH, Mannheim, Deutschland) nach den Angaben des Herstellers miteinander ligiert. Das Ligationsgemisch wurde durch Elektroporation in Corynebacterium glutamicum ATCC13032 transferiert. Die Selektion wurde auf LB-Agar mit 5 µg/ml Tetracyclin durchgeführt. Nach Inkubation für 48 Stunden bei 30°C wurden Kolonien isoliert, die den neuen Plasmidvektor enthielten. Die Anwesenheit des Plasmidvektors in den transformierten Bakterienzellen wurde durch eine alkalische Lysemethode ("QIAGEN Plasmid Mini Handbook for Plasmid Mini Kit", Qiagen GmbH, Hilden, Deutschland, 1997) nachgewiesen. Das isolierte Plasmid wurde pSELF1-1 genannt. Restriktionsanalysen von pSELF1-1 sowie ein Vergleich der erhaltenen Fragmentlängen mit DNA-Fragmenten bekannter Länge ergaben die Restriktionskarte, die als Abbildung 7 beigefügt ist.

Plasmid pSELF1-1 besteht durch diesen Konstruktionsweg ausschließlich aus DNA-Fragmenten, die aus Corynebacterium glutamicum stammen.

### Beispiel 11

### Produktion von Lysin unter Verwendung von pSELF1-1

Um die Kopienzahl eines Gens, das an der Biosynthese der Aminosäure Lysin in coryneformen Bakterien beteiligt ist, zu erhöhen, wurde das lysC(FBR)-Gen aus Corynebacterium glutamicum ausgewählt. Das lysC(FBR)-Gen kodiert eine gegen das Antimetabolit S-(2-Aminoethyl)-Cystein resistente Form des Enzyms Aspartatkinase und lag auf dem Plasmidvektor pJC30 kloniert vor (Cremer et al., Applied and Environmental Microbiology 57, 1746-1752 (1991)).

Zur Klonierung des lysC(FBR)-Gens in den in Beispiel 10 beschriebenen Plasmidvektor pSELF1-1, wurde Plasmid-DNA von pSELF1-1 und von pJC30 mit den Restriktionsenzymen EcoRI und ScaI (Pharmacia Biotech Europe GmbH, Freiburg, Deutschland) nach Herstellerangaben gespalten. Anschließend wurden die Restriktionsansätze mit dem Enzym T4 DNA-Ligase miteinander ligiert (Roche Diagnostics GmbH, Mannheim, Deutschland) und in den Bakterienstamm Corynebacterium glutamicum ATCC13032 transformiert. Die Selektion wurde auf LB-Agar mit 5 µg/ml Tetracyclin durchgeführt. Durch eine alkalische Lysemethode ("QIAGEN Plasmid Mini Handbook for Plasmid Mini Kit", Qiagen GmbH, Hilden, Deutschland, 1997) wurde aus transformierten Kolonien Plasmid-DNA reisoliert. Durch Restriktionsanalysen dieser Plasmid-DNA und durch Vergleiche mit DNA-Fragmenten bekannter Länge wurde das Plasmid pSELF1-lysC isoliert, das aus dem Plasmidvektor pSELF1-1 und dem lysC(FBR)-Genbereich besteht.

Die Plasmide pSELF1-lysC und der Kontrollvektor pSELF1-1 wurden per Elektroporation in den Stamm Corynebacterium glutamicum ATCC13032 transferiert. Der Plasmidtransfer wurde anschließend mittels alkalischer Lyse und Gelelektrophorese nachgewiesen ("QIAGEN Plasmid Mini Handbook for Plasmid Mini Kit", Qiagen GmbH, Hilden, Deutschland, 1997). Die auf diese Weise konstruierten Stämme ATCC13032 [pSELF1-1] und ATCC13032 [pSELF1-lysC] wurden zur Produktion von Lysin eingesetzt.

Die beiden Stämme wurden zunächst für 24 Stunden bei 30°C in 50 ml Luria-Bertani Medium mit 5 µg/ml Tetracyclin angezogen. Anschließend wurde jeweils 1 ml Kultur zweimal in Mineralmedium (Bröer et al., Applied and Environmental Microbiology 59, 316-321 (1993)) gewaschen, in 100 ml Mineralmedium mit 5 µg/ml Tetracyclin übertragen und erneut für 24 Stunden bei 30°C inkubiert. Je 5 ml Kulturüberstand wurden für 15 Minuten bei 13800xg und 4°C pelletiert und mit einer Millex-GS Filtereinheit (0,22 µm, Millipore S.A., Molsheim, Frankreich) sterilfiltiert. Die Bestimmung von Lysin in den filtrierten Kulturüberständen erfolgte mittels HPLC-Analytik nach der Methode von Büntemeyer et al. (Cytotechnology 5, 57-67 (1991)). Die erhaltenen Lysin-Konzentrationen nach 24 Stunden Kultur sind in Tabelle 2 zusammengefaßt.

**Tabelle 2**

| Lysin-Konzentration in Kulturüberständen von verschiedenen Stämmen von Corynebacterium glutamicum | | |
|---|---|---|
| Wirt | Plasmid | Lysin-Konzentration |
| | | (g / l) |
| ATCC13032 | pSELF1-1 | 0,02 |
| ATCC13032 | pSELF1-lysC | 1,0 |

### Beispiel 12

### Produktion von Pantothensäure unter Verwendung von pSELF3-1

Zur Erhöhung der Kopienzahl eines Gens, das an der Biosynthese von Pantothenat in coryneformen Bakterien beteiligt ist, wurde das panD-Gen aus Corynebacterium glutamicum ATCC13032 ausgewählt. Das panD-Gen kodiert für das Enzym L-Aspartat-α-Decarboxylase und lag auf dem Plasmidvektor pND10 kloniert vor (Dusch et al., Applied and Environmental Microbiology 65, 1530-1539 (1999)).

Zur Klonierung des panD-Gens in den neuen, in Beispiel 9 beschriebenen Plasmidvektor pSELF3-1, wurde Plasmid-DNA von pSELF3-1 mit den Restriktionsenzymen SacI (Pharmacia Biotech Europe GmbH, Freiburg, Deutschland) und BstZ17I (New England Biolabs GmbH, Schwalbach, Deutschland) und Plasmid-DNA von pND10 mit den Restriktionsenzymen SacI und ScaI (Pharmacia Biotech Europe GmbH, Freiburg, Deutschland) entsprechend den Herstellerangaben gespalten. Anschließend wurden die Restriktionsansätze mit dem Enzym T4 DNA-Ligase nach Herstellerangaben miteinander ligiert (Roche Diagnostics GmbH, Mannheim, Deutschland) und in den Bakterienstamm Corynebacterium glutamicum ATCC13032 transformiert. Die Selektion wurde auf LB-Agar mit 5 µg/ml Tetracyclin durchgeführt. Aus den transformierten Kolonien wurde durch alkalische Lyse Plasmid-DNA reisoliert ("QIAGEN Plasmid Mini Handbook for Plasmid Mini Kit", Qiagen GmbH, Hilden, Deutschland, 1997). Nach Restriktionsanalysen der isolierten Plasmid-DNA und durch einen Vergleich mit DNA-Fragmenten bekannter Länge (DNA Molecular Weight Marker X, Roche Diagnostics GmbH, Mannheim, Deutschland) wurde das Plasmid pSELF3-panD isoliert, das aus dem Plasmidvektor pSELF3-1 und dem für das panD-Gen kodierenden Bereich von pND10 besteht.

Zur Analyse der Pantothenat-Produktion in coryneformen Bakterien wurden der konstruierte Plasmidvektor pSELF3-panD und der Kontrollvektor pSELF3-1 in den Stamm ATCC13032ΔilvA (Sahm et al., Applied and Environmental Microbiology 65, 1973-1979 (1999)) transferiert. Das Vorhandensein der Plasmide wurde danach mittels alkalischer Lyse nachgewiesen ("QIAGEN Plasmid Mini Handbook for Plasmid Mini Kit", Qiagen GmbH, Hilden, Deutschland, 1997). Die auf diese Weise konstruierten Stämme ATCC13032ΔilvA [pSELF3-1] und ATCC13032ΔilvA [pSELF3-panD] wurden zur Produktion von Pantothenat eingesetzt.

Die Bakterienstämme wurden zunächst für 24 Stunden bei 30°C in 50 ml Luria-Bertani Medium mit 5 µg/ml Tetracyclin angezogen. Anschließend wurde 1 ml der Bakterienkultur zweimal mit CGXII-Medium (Keilhauer et al., Journal of Bacteriology 175, 5595-5603, (1993)), dem 2 mM Isoleucin (Sigma-Aldrich Chemie GmbH, Deisenhofen, Deutschland) zugesetzt wurden, gewaschen, in 50 ml CGXII-Medium mit 2 mM Isoleucin und 5 µg/ml Tetracyclin übertragen und für 24 Stunden bei 30°C angezogen. Mit 3 ml dieser Kultur wurden erneut 50 ml CGXII-Medium, das 2 mM Isoleucin enthielt, inokuliert. Nach weiterer Inkubation des Ansatzes für 24 Stunden bei 30°C wurden 20 ml der Bakterienkultur für 10 Minuten mit 1250xg pelletiert. Der Kulturüberstand wurde anschließend mit einer Millex-GS Filtereinheit (0,22 µm, Millipore S.A., Molsheim, Frankreich) sterilfiltiert. Die Bestimmung der Pantothenat-Konzentration in den filtrierten Kulturüberständen wurde nach der Anleitung des Difco-Manual, 10^{th} Edition (Difco Laboratories, Detroit, Michigan, USA) durchgeführt. Die erhaltenen Pantothenat-Konzentrationen nach 24 Stunden Kultur sind in Tabelle 3 zusammengefaßt.

**Tabelle 3**

| Pantothenat-Konzentration in Kulturüberständen von verschiedenen Stämmen von Corynebacterium glutamicum | | |
|---|---|---|
| Wirt | Plasmid | Pantothenat-Konzentration |
| | | (ng / ml) |
| ATCC13032ΔilvA | pSELF3-1 | 14,1 |
| ATCC13032ΔilvA | pSELF3-panD | 54,1 |

Weiterhin wurde der konstruierte Plasmidvektor pSELF3-panD eingesetzt, um den Stamm ATCC13032ΔilvA [pEKEx2panBC, pECM3ilvBNCD] (Sahm et al., Applied and Environmental Microbiology 65, 1973-1979 (1999)) weiter zu verbessern. Dieser Stamm trägt auf bekannten Plasmidvektoren bereits die Gene ilvBNCD und panBC, die sich vorteilhaft auf die Biosynthese von Pantothenat auswirken.

Der Plasmidvektor pSELF3-panD und der Kontrollvektor pSELF3-1 wurden durch Elektroporation in den Stamm ATCC13032ΔilvA [pEKEx2panBC, pECM3ilvBNCD] (Sahm et al., Applied and Environmental Microbiology 65, 1973-1979 (1999)) transferiert. Die Selektion erfolgte auf LB-Agar mit 5 µg/ml Tetracyclin. Das Vorhandensein der transferierten Plasmidvektoren und der bereits im Bakterienstamm vorliegenden Plasmide wurde danach mittels alkalischer Lyse nachgewiesen ("QIAGEN Plasmid Mini Handbook for Plasmid Mini Kit", Qiagen GmbH, Hilden, Deutschland, 1997). Beide auf diese Weise konstruierten Stämme wurden ebenfalls in der oben beschriebenen Weise zur Produktion von Pantothenat eingesetzt. Die erhaltenen Pantothenat-Konzentrationen in den Kulturüberständen nach 24 Stunden Kultur sind in Tabelle 4 dargestellt.

**Tabelle 4**

| Pantothenat-Konzentration in Kulturüberständen von verschiedenen Stämmem von Corynebacterium glutamicum | | |
|---|---|---|
| Wirt | Plasmide | Pantothenat-Konzentration |
| | | (ng / ml) |
| ATCC13032ΔilvA | pECM3ilvBNCD pEKEx2panBC pSELF3-1 | 18,3 |
| ATCC13032ΔilvA | pECM3ilvBNCD pEKEx2panBC pSELF3-panD | 655,2 |

Folgende Abbildungen sind beigefügt:
- Abbildung 1:Restriktionskarte des Plasmids pTET3.
- Abbildung 2:Restriktionskarte des Plasmids pCRY4.
- Abbildung 3: Karte der Replikationsregion des Plasmids pTET3.
- Abbildung 4:Karte der Replikationsregion des Plasmids pCRY4
- Abbildung 5: Karte der Antibiotikaresistenzregion des Plasmids pTET3.
- Abbildung 6: Karte des Plasmidvektors pSELF3-1.
- Abbildung 7:Karte des Plasmidvektors pSELF1-1.

Längenangaben sind als ca.-Angaben aufzufassen. Die verwendeten Abkürzungen und Bezeichnungen haben folgende Bedeutung:
- bps:: Basenpaare
- AvrII:: Schnittstelle für das Restriktionsenzym AvrII
- ClaI:: Schnittstelle für das Restriktionsenzym ClaI
- EcoRI:: Schnittstelle für das Restriktionsenzym EcoRI
- EcoRV:: Schnittstelle für das Restriktionsenzym EcoRV
- FspI:: Schnittstelle für das Restriktionsenzym FspI
- HindIII:: Schnittstelle für das Restriktionsenzym HindIII
- HpaI:: Schnittstelle für das Restriktionsenzym HpaI
- MunI:: Schnittstelle für das Restriktionsenzym MunI
- NruI:: Schnittstelle für das Restriktionsenzym NruI
- PstI:: Schnittstelle für das Restriktionsenzym PstI
- SacI:: Schnittstelle für das Restriktionsenzym SacI
- SacII:: Schnittstelle für das Restriktionsenzym SacII
- SalI:: Schnittstelle für das Restriktionsenzym SalI
- ScaI:: Schnittstelle für das Restriktionsenzym ScaI
- SmaI:: Schnittstelle für das Restriktionsenzym SmaI
- SpeI:: Schnittstelle für das Restriktionsenzym SpeI
- SphI:: Schnittstelle für das Restriktionsenzym SphI
- XbaI:: Schnittstelle für das Restriktionsenzym XbaI
- XhoI:: Schnittstelle für das Restriktionsenzym XhoI
- aadA:: Gen für das Spectinomycin-Streptomycin-Resistenzprotein
- parA:: Gen für das Stabilisierungsprotein ParA
- sulI:: Gen für das Sulfamethoxazol-Resistenzprotein
- repA:: Gen für das Replikationsprotein RepA
- tetA:: Gen für das Tetracyclin-Resistenzprotein
- tetR:: Gen für das Tetracyclin-Repressorprotein

## Patentansprüche

1. Miteinander kompatible Plasmide pTET3 und pCRY4, isoliert aus dem unter der Nummer DSM 5816 hinterlegten Stamm von Corynebacterium glutamicum, wobei das Plasmid pTET3 charakterisiert ist durch
1.1 eine Länge von ~ 27,8 kbp und die in Abbildung 1 dargestellte Restriktionskarte,
1.2 eine Replikationsregion mit der Nukleotidsequenz, dargestellt in SEQ ID No. 1, und eine
1.3 Antibiotikaresistenzregion, bestehend aus einem Tetracyclinresistenz-vermittelnden tetA-Gen und einem Streptomycin- und Spectinomycinresistenzvermittelnden aadA-Gen, dargestellt in SEQ ID No. 6,
und das Plasmid pCRY4 charakterisiert ist durch
1.4 eine Länge von ~ 48 kbp und die in Abbildung 2 dargestellten Restriktionskarte und
1.5 eine Replikationsregion mit der Nukleotidsequenz, dargestellt in SEQ ID No. 4.

2. Zur autonomen Replikation in coryneformen Bakterien befähigte zusammengesetzte Plasmide enthaltend
2.1 zumindest eine DNA-Replikationsregion, von einem der Plasmide pTET3 gemäß Anspruch 1.2 oder pCRY4 gemäß Anspruch 1.5
2.2 ein Genfragment von einem Plasmid aus E. coli, B. subtilis oder Streptomyces, das sich in den genannten Mikroorganismen vermehren kann, und
2.3 mindestens eine Region für die Expression einer Wirkstoffresistenz.

3. Zusammengesetzte Plasmide gemäß Anspruch 2,
die die Wirkstoffresistenz(en) aus dem Plasmid pTET3 gemäß Anspruch 1 enthalten.

4. Zusammengesetzte Plasmide gemäß Anspruch 3,
die Teile oder die Gesamtmenge der Nukleotidsequenzen der Plasmide pGA1 und/oder pGA2 enthalten., welche aus DSM5816 isolierbar sind.

5. Zusammengesetzte Plasmide gemäß Anspruch 2,
die mindestens ein DNA-Fragment enthalten, das für ein Gen aus dem Biosyntheseweg eines Vitamins, eines Nukleotids oder einer L-Aminosäure kodiert und in coryneformen Bakterien exprimiert wird.

6. Zur autonomen Replikation in coryneformen Bakterien befähigte Plasmidvektoren, enthaltend
6.1 zumindest eine Replikationsregion, von einem der Plasmide pGA1, pGA2, welche aus DSM 5816 isolierbar sind, pTET3 gemäß Anspruch 1.2 oder pCRY4, gemäß Anspruch 1.5
6.2 mindestens eine Wirkstoffresistenz aus dem Plasmid pTET3 gemäß Anspruch 1.3 und gegebenenfalls
6.3 mindestens ein DNA-Fragment, das für ein Gen aus dem Biosyntheseweg eines Vitamins, eines Nukleotids oder einer L-Aminosäure kodiert und in coryneformen Bakterien exprimiert wird.

7. Plasmidvektor pSELF3-1,
**dadurch gekennzeichnet, dass** er
a) eine Länge von ~ 7,0 kbp aufweist
b) die Replikationsregion des Plasmids pTET3 gemäß Anspruch 1.2 enthält
c) das Tetracyclinresistenz vermittelnde tetA-Gen gemäß Anspruch 1.3 enthält, und
d) die Restriktionskarte gemäß Abbildung 6 aufweist.

8. Plasmidvektor pSELF1-1,
**dadurch gekennzeichnet, dass** er
a) eine Länge von ~ 7,3 kbp aufweist,
b) das Plasmid pGA1, isolierbar aus DSM 5816, enthält
c) das Tetracyclinresistenz vermittelnde tetA-Gen gemäß Anspruch 1.3 enthält, und
d) die Restriktionskarte gemäß Abbildung 7 aufweist.

9. Verfahren zur Herstellung von L-Aminosäuren, insbesondere L-Lysin oder L-Threonin, durch Fermentation von coryneformen Bakterien,
**dadurch gekennzeichnet,**
**daß** man Stämme einsetzt, in denen ein oder mehrere Plasmidvektor(en) gemäß den Ansprüchen 2 bis 7 enthalten sind (ist).

10. Verfahren zur Herstellung von Vitaminen, insbesondere D-Pantothensäure durch Fermentation von coryneformen Bakterien,
**dadurch gekennzeichnet,**
**daß** man Stämme einsetzt, in denen ein oder mehrere Plasmidvektor(en) gemäß den Ansprüchen 7 und 8 enthalten sind (ist).

11. DNA Fragment kodierend für das Stabilisierungsprotein ParA des Plasmids pTET3 mit der Aminosäuresequenz von SEQ ID No. 2.

12. Das DNA-Fragment gemäß Anspruch 11, **dadurch gekennzeichnet, dass** es die Nukleotidsequenz von Position 228 bis 824 von SEQ ID NO: 1 umfasst.

13. DNA-Fragment kodierend für das Replikationsprotein RepA des Plasmids pTET3 mit der Aminosäuresequenz von SEQ ID NO: 3.

14. Das DNA-Fragment gemäß Anspruch 13, **dadurch gekennzeichnet, dass** es die Nukleotidsequenz von Position 1829 bis 3295 von SEQ ID NO: 1 umfasst.

15. Das DNA-Fragment gemäß einem oder mehreren der Ansprüche 11-14 dargestellt in SEQ ID NO: 1.

16. DNA-Fragment kodierend für das Replikationsprotein repA des Plasmids pCRY4 mit der Aminosäuresequenz von SEQ ID NO: 5.

17. Das DNA-Fragment gemäß Anspruch 16, **dadurch gekennzeichnet, dass** es die Nukleotidsequenz von Position 338 bis 1291 von SEQ ID NO: 4 umfasst.

18. Das DNA-Fragment gemäß Anspruch 17, das die Nukleotidsequenz von SEQ ID NO: 4 umfasst.

19. DNA-Fragment kodierend für das Tetracyclin-Resistenzprotein TetA mit der Aminosäuresequenz von SEQ ID NO: 7.

20. Das DNA-Fragment gemäß Anspruch 19, **dadurch gekennzeichnet, dass** es die Nukleotidsequenz von Position 2124 bis 3272 von SEQ ID NO: 6 umfasst.

21. DNA-Fragment kodierend für das Spectinomycin/Streptomycin-Resistenzprotein mit der Aminosäuresequenz von SEQ ID NO: 8.

22. Das DNA-Fragment gemäß Anspruch 21, **dadurch gekennzeichnet, dass** es die Nukleotidsequenz von Position 5882 bis 6718 von SEQ ID NO: 6 umfasst.

23. Das DNA-Fragment gemäß einem oder mehreren der Ansprüche 19 bis 22, **dadurch gekennzeichnet, dass** es die Nukleotidsequenz von SEQ ID NO: 6 umfasst.

## Claims

1. Mutually compatible plasmids pTET3 and pCRY4, isolated from the Corynebacterium glutamicum strain deposited under DSM 5816, wherein the pTET3 plasmid is **characterized by**
1.1. a length of ~ 27.8 kbp and the restriction map depicted in Figure 1,
1.2. a replication region having the nucleotide sequence depicted in SEQ ID No. 1, and an
1.3. antibiotic resistance region consisting of a tetracycline resistance-mediating tetA gene and a streptomycin resistance and spectinomycin resistance-mediating aadA gene, depicted in SEQ ID No. 6,
and the pCRY4 plasmid is **characterized by**
1.4. a length of ~ 48 kbp and the restriction map depicted in Figure 2,
1.5. a replication region having the nucleotide sequence depicted in SEQ ID No. 4.

2. Composite plasmids capable of autonomous replication in coryneform bacteria, comprising
2.1. at least one DNA replication region of either of the plasmids pTET3 according to Claim 1.2 and pCRY4 according to Claim 1.5,
2.2. a gene fragment of a plasmid from E. coli, B. subtilis or Streptomyces, which gene fragment can propagate in said microorganisms, and
2.3. at least one region for expressing a drug resistance.

3. Composite plasmids according to Claim 2, which comprise the drug resistance(s) from the pTET3 plasmid according to Claim 1.

4. Composite plasmids according to Claim 3, which comprise parts or all of the nucleotide sequences of the plasmids pGA1 and/or pGA2 which can be isolated from DSM5816.

5. Composite plasmids according to Claim 2, which comprise at least one DNA fragment which codes for a gene of the biosynthetic pathway of a vitamin, a nucleotide or an L-amino acid and which is expressed in coryneform bacteria.

6. Plasmid vectors capable of autonomous replication in coryneform bacteria, comprising
6.1. at least one replication region of any of the plasmids pGA1, pGA2 which can be isolated from DSM 5816, pTET3 according to Claim 1.2, or pCRY4 according to Claim 1.5,
6.2. at least one drug resistance from the pTET3 plasmid according to Claim 1.3, and, where appropriate,
6.3. at least one DNA fragment which codes for a gene of the biosynthetic pathway of a vitamin, a nucleotide or an L-amino acid and which is expressed in coryneform bacteria.

7. Plasmid vector pSELF3-1, **characterized in that** it
a) has a length of ~ 7.0 kbp,
b) comprises the replication region of the pTET3 plasmid according to Claim 1.2,
c) comprises the tetracycline resistance-mediating tetA gene according to Claim 1.3, and
d) has the restriction map according to Figure 6.

8. Plasmid vector pSELFl-1, **characterized in that** it
a) has a length of ~ 7.3 kbp,
b) comprises the pGA1 plasmid which can be isolated from DSM 5816,
c) comprises the tetracycline resistance-mediating tetA gene according to Claim 1.3, and
d) has the restriction map according to Figure 7.

9. Method for producing L-amino acids, in particular L-lysine or L-threonine, by fermentation of coryneform bacteria, **characterized in that**
strains are used which harbour one or more plasmid vector(s) according to Claims 2 to 7.

10. Method for producing vitamins, in particular D-pantothenic acid, by fermentation of coryneform bacteria, **characterized in that**
strains are used which harbour one or more plasmid vector(s) according to Claims 7 and 8.

11. DNA fragment coding for the ParA stabilization protein of the pTET3 plasmid, having the amino acid sequence of SEQ ID No. 2.

12. DNA fragment according to Claim 11, **characterized in that** it comprises the nucleotide sequence from position 228 to position 824 of SEQ ID NO: 1.

13. DNA fragment coding for the RepA replication protein of the pTET3 plasmid, having the amino acid sequence of SEQ ID NO: 3.

14. DNA fragment according to Claim 13, **characterized in that** it comprises the nucleotide sequence from position 1829 to position 3295 of SEQ ID NO: 1.

15. DNA fragment according to one or more of Claims 11-14, depicted in SEQ ID NO: 1.

16. DNA fragment coding for the repA replication protein of the pCRY4 plasmid, having the amino acid sequence of SEQ ID NO: 5.

17. DNA fragment according to Claim 16, **characterized in that** it comprises the nucleotide sequence from position 338 to position 1291 of SEQ ID NO: 4.

18. DNA fragment according to Claim 17, which comprises the nucleotide sequence of SEQ ID NO: 4.

19. DNA fragment coding for the TetA tetracycline resistance protein having the amino acid sequence of SEQ ID NO: 7.

20. DNA fragment according to Claim 19, **characterized in that** it comprises the nucleotide sequence from position 2124 to position 3272 of SEQ ID NO: 6.

21. DNA fragment coding for the spectinomycin/streptomycin resistance protein having the amino acid sequence of SEQ ID NO: 8.

22. DNA fragment according to Claim 21, **characterized in that** it comprises the nucleotide sequence from position 5882 to position 6718 of SEQ ID NO: 6.

23. DNA fragment according to one or more of Claims 19 to 22, **characterized in that** it comprises the nucleotide sequence of SEQ ID NO: 6.

## Revendications

1. Plasmides compatibles les uns avec les autres pTET3 et pCRY4, isolés de la souche de Corynebacterium glutamicum enregistrée sous le numéro DSM 5816, le plasmide pTET3 étant **caractérisé par** :
1.1 une longueur de ~ 27,8 kbp et la carte de restriction représentée à la Fig. 1,
1.2 une région de réplication avec la séquence de nucléotides, représentée à la SEQ ID n° 1, et une
1.3 région de résistance aux antibiotiques constituée d'un gène tetA agent de résistance à la tétracycline et un gène aadA agent de résistance à la streptomycine et spectinomycine, représenté à la SEQ ID n° 6,
et le plasmide pCRY4 étant **caractérisé par** :
1.4 une longueur de ~ 48 kbp et la carte de restriction représentée à la Fig. 2, et
1.5 une région de réplication avec la séquence de nucléotides, représentée à la SEQ ID n°4.

2. Plasmides composés capables d'une réplication autonome dans des bactéries coryneformes, contenant :
2.1 au moins une région de réplication d'ADN d'un des plasmides pTET3 selon la revendication 1.2 ou pCRY4 selon la revendication 1.5
2.2 un fragment génétique d'un plasmide d'E. coli, B. subtilis ou Streptomycète qui peut se multiplier dans les microorganismes susmentionnés, et
2.3 au moins une région pour l'expression d'une résistance aux principes actifs.

3. Plasmides composés selon la revendication 2, qui contiennent la/les résistance/s aux principes actifs provenant du plasmide pTET3 selon la revendication 1.

4. Plasmides composés selon la revendication 3, qui contiennent des parties ou la quantité totale des séquences de nucléotides des plasmides pGA1 et/ou pGA2, lesquels sont isolables à partir du DSM5816.

5. Plasmides composés selon la revendication 2, contenant au moins un fragment d'ADN qui code un gène de la voie de biosynthèse d'une vitamine, d'un nucléotide ou d'un acide L-aminé et qui est exprimé dans des bactéries coryneformes.

6. Vecteurs plasmidiques capables d'une réplication autonome dans des bactéries coryneformes, contenant :
6.1 au moins une région de réplication d'un des plasmides pGA1, pGA2, lesquels sont isolables à partir du DSM 5816, pTET3 selon la revendication 1.2 ou pCRY4 selon la revendication 1.5
6.2 au moins une résistance aux principes actifs du plasmide pTET3 selon la revendication 1.3, et éventuellement
6.3 au moins un fragment d'ADN qui code un gène de la voie de biosynthèse d'une vitamine, d'un nucléotide ou d'un acide L-aminé et est exprimé dans des bactéries coryneformes.

7. Vecteur plasmidique pSELF3-1, **caractérisé en ce qu'**il :
a) présente une longueur de ~ 7,0 kbp,
b) contient la région de réplication du plasmide pTET3 selon la revendication 1.2
c) contient le gène tetA agent de la résistance à la tétracycline selon la revendication 1.3, et
d) présente la carte de restriction selon la Fig. 6.

8. Vecteur plasmidique pSELF1-1, **caractérisé en ce qu'**il :
a) présente une longueur de ~ 7,3 kbp,
b) contient le plasmide pGA1, isolable à partir du DSM 5816,
c) contient le gène tetA agent de la résistance à la tétracycline selon la revendication 1.3, et
d) présente la carte de restriction selon la Fig. 7.

9. Procédé de préparation d'acides L-aminés, en particulier de L-lysine ou de L-thréonine, par fermentation de bactéries coryneformes, **caractérisé en ce qu'**on utilise des souches dans lesquelles sont contenus un ou plusieurs vecteur/s plasmidique/s selon les revendications 2 à 7.

10. Procédé de préparation de vitamines, en particulier d'acide D-pantothénique par fermentation de bactéries coryneformes, **caractérisé en ce qu'**on utilise des souches dans lesquelles sont contenus un ou plusieurs vecteur/s plasmidique/s selon les revendications 7 et 8.

11. Fragment d'ADN codant la protéine de stabilisation ParA du plasmide pTET3 avec la séquence d'acides aminés de la SEQ ID n° 2.

12. Fragment d'ADN selon la revendication 11, **caractérisé en ce qu'**il comprend la séquence de nucléotides de la position 228 à 824 de la SEQ ID n° 1.

13. Fragment d'ADN codant la protéine de réplication RepA du plasmide pTET3 avec la séquence d'acides aminés de la SEQ ID n° 3.

14. Fragment d'ADN selon la revendication 13, **caractérisé en ce qu'**il comprend la séquence de nucléotides de la position 1829 à 3295 de la SEQ ID n° 1.

15. Fragment d'ADN selon l'une ou plusieurs des revendications 11-14 représenté à la SEQ ID n° 1.

16. Fragment d'ADN codant la protéine de réplication repA du plasmide pCRY4 avec la séquence d'acides aminés de SEQ ID n° 5.

17. Fragment d'ADN selon la revendication 16, **caractérisé en ce qu'**il comprend la séquence de nucléotides de la position 338 à 1291 de la SEQ ID n° 4.

18. Fragment d'ADN selon la revendication 17 qui comprend la séquence de nucléotides de la SEQ ID n° 4.

19. Fragment d'ADN codant la protéine de résistance à la tétracycline TetA avec la séquence d'acides aminés de la SEQ ID n° 7.

20. Fragment d'ADN selon la revendication 19, **caractérisé en ce qu'**il comprend la séquence de nucléotides de la position 2124 à 3272 de la SEQ ID n° 6.

21. Fragment d'ADN codant la protéine de résistance à la spectinomycine/streptomycine avec la séquence d'acides aminés de la SEQ ID n° 8.

22. Fragment d'ADN selon la revendication 21, **caractérisé en ce qu'**il comprend la séquence de nucléotides de la position 5882 à 6718 de la SEQ ID n° 6.

23. Fragment d'ADN selon l'une ou plusieurs des revendications 19 à 22, **caractérisé en ce qu'**il comprend la séquence de nucléotides de la SEQ ID n° 6.
